(19) Europäisches Patentamt
European Patent Office
Office européen des brevets

(11) EP 1 832 659 A1

(12) EUROPEAN PATENT APPLICATION
published in accordance with Art. 158(3) EPC

(43) Date of publication:
**12.09.2007 Bulletin 2007/37**

(21) Application number: **05844853.1**

(22) Date of filing: **28.12.2005**

(51) Int Cl.:
***C12P 19/22*** *(2006.01)*

(86) International application number:
**PCT/JP2005/024113**

(87) International publication number:
**WO 2006/070883 (06.07.2006 Gazette 2006/27)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **28.12.2004 JP 2004381780**

(71) Applicants:
- **Suntory Logistics Limited**
**Osaka-shi,**
**Osaka 530-8203 (JP)**
- **SAN-EI GEN F.F.I., INC.**
**Toyonaka-shi,**
**Osaka 561-8588 (JP)**

(72) Inventors:
- **Ono, Yoshiko,**
**Suntroy Limited Research Center**
**Osaka 6188503 (JP)**
- **Tomimori, Namino,**
**Suntory Limited Research Center**
**Osaka, 6188503 (JP)**
- **Tateishi, Norifumi**
**Suntory Limited Research Center**
**Osaka 6188503 (JP)**
- **Moriwaki, Masamitsu,**
**SAN-EI GEN F.F.I., Inc.**
**Osaka, 5618588 (JP)**
- **Emura, Kazuhiro,**
**SAN-EI GEN F.F.I., Inc.**
**Osaka, 5618588 (JP)**
- **Okuyama, Shuji,**
**SAN-EI GEN F.F.I., Inc.**
**Osaka, 5618588 (JP)**

(74) Representative: **Vossius & Partner**
**Siebertstrasse 4**
**81675 München (DE)**

# (54) QUERCETIN GLYCOSIDE COMPOSITION AND METHOD OF PREPARING THE SAME

(57) The present invention provides an α-glycosyl isoquercitrin-containing novel composition which has a high in vivo absorbability, and hence exhibits a significant in vivo antioxidative activity. The present invention further provides preparation methods for such a composition. The composition contains a mixture of quercetin glycosides represented by the following formula:

[Formula 1]

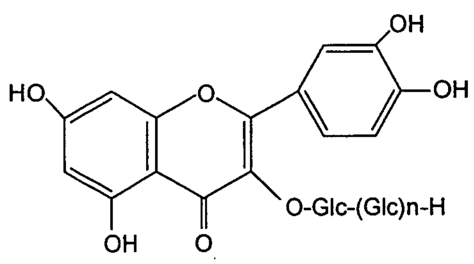

wherein Glc represents a glucose residue, and n is 0 or a positive integer of 1 or more, includes at least a quercetin glycoside wherein n is 3, and satisfies the following requirement (a):
(a) the total proportion of quercetin glycosides in which n is 3, and in which other n values may be 1 or 2, or 1 and 2, is 50 mol% or more, and the total proportion of quercetin glycosides wherein n is 4 or more is 15 mol% or less, in the composition. The composition can be prepared by treating an enzymatically modified isoquercitrin with β-amylase.

EP 1 832 659 A1

FIG. 6

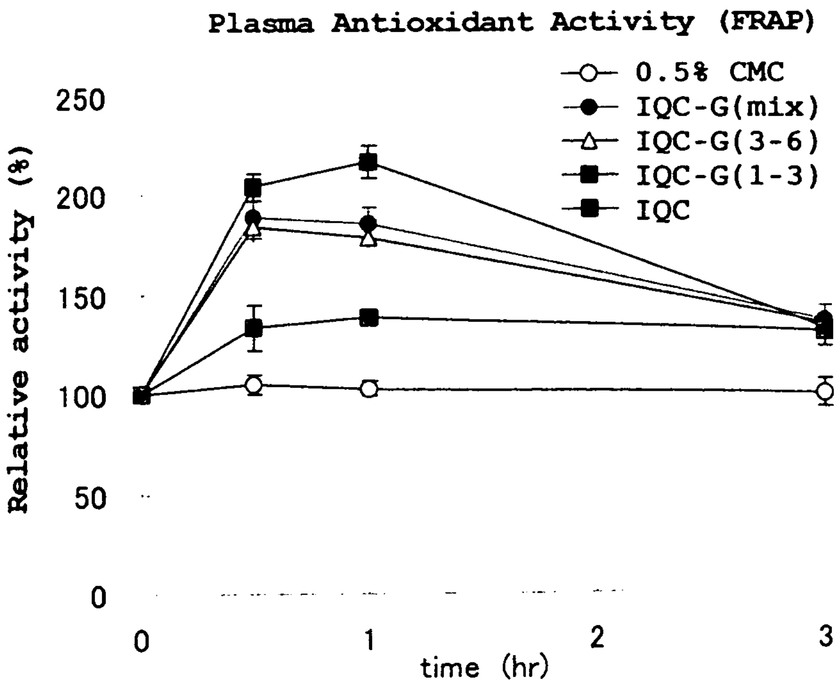
Plasma Antioxidant Activity (FRAP)
0.5% CMC
IQC-G(mix)
IQC-G(3-6)
IQC-G(1-3)
IQC
Relative activity (%)
250
200
150
100
50
0
0
1
2
3
time (hr)

**Description**

TECHNICAL FIELD

The present invention relates to novel compositions comprising a mixture of isoquercitrin and α-glycosyl isoquercitrin (hereinafter generally referred to as "quercetin glycosides"), widely used in the fields of food products, cosmetic materials, etc., as antioxidants, anti-fading agents, flavor-change inhibitors, etc. The present invention also relates to methods for preparing the compositions. The compositions of the present invention are significant in orally administered in vivo absorbability and anti-oxidative activity, and hence preferably usable as antioxidants for the living body.

BACKGROUND ART

Lately, it has become known that oxidative stress induced by reactive oxygen species and free radicals causes various diseases including lifestyle-related diseases. It is a fact that oxygen is a quintessential molecular for producing energy to sustain life, while excessive oxygen transforms to extremely reactive oxygen and damages the living body. Reactive oxygen species include superoxide anion radicals ($\cdot O_2^-$), hydrogen peroxide ($H_2O_2$), OH radicals ($\cdot OH$) and single oxygen ($^1O_2$), excited molecular species, etc. Living organisms are inherently able to prevent oxidative disorders caused by reactive oxygen species, for which vitamin E and anti-oxidase are responsible. However, when the ability to defend against oxidative disorders is suppressed due to factors such as aging, etc., or when an amount of reactive oxygen species is generated that exceeds the amount the body can defend against due to factors such as intense exercise, stress, etc., the reactive oxygen species, which are left unmediated, oxidize target molecules. As a result, living components are damaged, and aging is induced.

Consequently, it is thought to be important to efficiently take anti-oxidative substances, which mediate reactive oxygen species and free radicals, and defend against oxidative stress when considering the prevention and treatment of various diseases. In particular, since oxidative disorders can presumably be controlled and mediated more efficiently by aggressively increasing intake of defensive mechanism components against oxidative disorders from food, a wide variety of food components with anti-oxidative effects are drawing much attention.

Flavonoids are contained in everyday food in many different forms, and are known to have strong anti-oxidative activities. However, flavonoids with anti-oxidative properties have low orally administered in vivo absorbability and are hence not good enough to mediate reactive oxygen species and free radicals in vivo, despite their ex vivo effectiveness. A method then proposed is to bind glucose to flavonoids (hesperidin, diosmin, naringin, neohesperidin) to enhance absorbability (see Patent document 1). Further, it is reported that α-glycosyl rutin obtained by the glucose transfer to rutin contained in buckwheat, etc., has more improved absorbability compared with rutin (see Patent document 2 and Non-patent document 1).

Quercetin (Quercetin: 3,3',4',5,7-pentahydroxyflavone), aglycone of rutin, is known to have versatile physiology such as platelet anti-aggregant and anti-adhesion effects, a vasodilating effect, anticarcinogenic activity, etc., in addition to strong anti-oxidative activities (see Non-patent document 2). Even for this quercetin, it is reported that quercetin glycosides (Quercetin-4'-β-D-glucoside and Quercetin-3,4'-β-D-glucoside) abundant in onions have higher absorbability (see Non-patent document 3). Similarly, it is reported that isoquercitrin, wherein glucose is bound to the third position of quercetin (Quercetin-3-β-D-glucoside), has higher absorbability than quercetin and rutin (see Non-patent document 4).

Isoquercitrin is a substance having higher absorbability than quercetin and rutin as mentioned above. However, due to its water-insolubility, it poses a problem as being only of limited use in water-based compositions such as food, beverages, etc. To solve this problem, a method is proposed to prepare α-glycosyl isoquercitrin by transferring a glucose residue of a substrate to a glucose residue site of isoquercitrin, using a glycosyltransferase (see Patent document 3). The thus prepared α-glycosyl isoquercitrin maintains the properties of isoquercitrin, but is an easily water soluble substance whose water solubility is improved. The substance is marketed under commercial names "SANMELIN® AO-1007" and "SANMELIN®powder C-10" as antioxidants (food additives) from San-Ei Gen F.F.I., INC.

Patent document 1: Unexamined Japanese Patent Publication No. 2000-78956
Patent document 2: Unexamined Japanese Patent Publication No. 2004-59522
Patent document 3: Unexamined Japanese Patent Publication No. 1989-213293
Non-patent document 1: Shimoi K. et al., J. Agric. Food Chem., 51, 2785-2789, 2003
Non-patent document 2: Middlton EJ. et al., Pharmacol. Rev., 52, 673-751, 2000
Non-patent document 3: Hollman PC. et al. Arch Toxicol Suppl., 20, 237-248, 1998
Non-patent document 4: Morand C. et al., Free Rad Res., 33, 667-676, 2000

DISCLOSURE OF THE INVENTION

PROBLEMS TO BE SOLVED BY THE INVENTION

As mentioned above, the enhancement of orally administered in vivo absorbability by glycosidation is evident in many flavonoids; however, their effects cannot yet be said to be sufficient.

An object of the present invention is hence to enhance orally administered in vivo absorbability of quercetin glycosides such as isoquercitrin and α-glycosyl isoquercitrin, kinds of flavonoids. More specifically, the object of the present invention is to provide a novel composition comprising a mixture of quercetin glycosides with improved orally administered in vivo absorbability. Another object of the present invention is to provide a method for preparing the novel composition. A further object of the present invention is to provide a method for enhancing the orally administered in vivo absorbability of quercetin glycoside compositions to be higher compared to that of conventional enzymatically modified isoquercitrin.

MEANS FOR SOLVING THE PROBLEMS

The present inventors conducted extensive studies to solve the above problem, and found that, depending on the number of glucose residues (n) binding to the α-position of a glucose residue of quercetin glycosides (Gn) represented by the following formula:

[Formula 1]

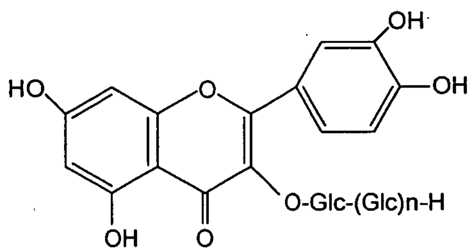

wherein Glc represents a glucose residue; and n is 0 or a positive integer of 1 or more,
there are differences in orally administered in vivo absorbability of quercetin glycosides (Gn). More specifically, mixtures of the above isoquercitrin wherein the number of glucose residues (n) is 0 (G0) and the above α-glycosyl isoquercitrin wherein the number of glucose residues (n) ranges from 1 to 7 (G1, G2,..., and G7) were examined for orally administered in vivo absorbability by partially collecting fractions abundant in G0, G1, G2, G3 or G4. As a result, the inventors surprisingly found that the mixtures containing abundant G3 had the highest orally administered in vivo absorbability, i.e., as the number of glucose residues (n) increases from 1, 2 to 3, the higher the orally administered in vivo absorbability became, and the orally administered in vivo absorbability diminishes when the number of glucose residues (n) is 4.

The present inventors continued further studies, and found that orally administered in vivo absorbability can be improved by decreasing the content (i.e. proportion) of α-glycosyl isoquercitrin wherein the number of glucose residues (n) is 4 or more (G(4≤)) in a mixture of quercetin glycosides, and that orally administered in vivo absorbability is further enhanced by reducing the content (i.e. proportion) of isoquercitrin wherein the number of glucose residues (n) is 0 (G0) in a mixture of quercetin glycosides. Furthermore, the present inventors verified that compositions (mixtures of quercetin glycosides) containing a large amount of the above α-glycosyl isoquercitrin wherein the number of glucose residues (n) ranges from 1 to 3 (G1 to G3), and a small amount of α-glycosyl isoquercitrin wherein the number of glucose residues is 4 or more (G(4≤)) and/or isoquercitrin wherein n is 0 (G0) have higher orally administered in vivo absorbability than conventionally known enzymatically modified isoquercitrin, and hence exhibit excellent in vivo antioxidative effects.

The present inventors also found that such compositions can be comparatively easily and stably prepared by treating an enzymatically modified isoquercitrin (isoquercitrin glycoside) with amylase, particularly with β-amylase, and that the above quercetin glycoside compositions having excellent orally administered in vivo absorbability can be industrially mass-produced.

More specifically, the present invention has the following aspects.

(1) Quercetin Glycoside Composition

(1-1) A quercetin glycoside composition comprising a mixture of quercetin glycosides represented by the following formula:

[Formula 2]

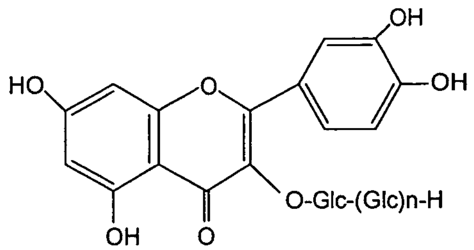

wherein Glc represents a glucose residue; and n is 0 or a positive integer of 1 or more,
the quercetin glycoside composition comprising at least a quercetin glycoside in which n is 3, and satisfying the following requirement (a):

(a) the composition comprises a mixture of quercetin glycosides in which n is 3, and in which other n values may be 1 or 2, or 1 and 2, in a total proportion of 50 mol% or more, and quercetin glycosides in which n is 4 or more in a total proportion of 15 mol% or less.

(1-2) The quercetin glycoside composition of (1-1), wherein the total proportion of quercetin glycosides in which n is 4 or more is 10 mol% or less.
(1-3) The quercetin glycoside composition of (1-1) or (1-2), wherein the total proportion of quercetin glycosides in which n is 3, and in which other n values may be 1 or 2, or 1 and 2, is 60 mol% or more.
(1-4) The quercetin glycoside composition of (1-1) or (1-2), wherein the total proportion of quercetin glycosides in which n is 3, and in which other n values may be 1 or 2, or 1 and 2, is 70 mol% or more.
(1-5) The quercetin glycoside composition of any one of (1-1) to (1-4), further satisfying at least one of the following requirements (b) and (c):

(b) the composition contains a quercetin glycoside in which n is 0 in 20 mol% or less, and
(c) the composition comprises a mixture of 2 types of quercetin glycosides, one in which n is 2, and one in which n is 3, and the total proportion thereof is 50 mol% or more.

(1-6) The quercetin glycoside composition of any one of (1-1) to (1-5), further satisfying the following requirement (d):

(d) the composition comprises a mixture of quercetin glycosides in which n is 3, and in which other n values may be 1 or 2, or 1 and 2, in the total proportion of 60 mol% or more, and a quercetin glycoside in which n is 0 in 20 mol% or less.

(1-7) The quercetin glycoside composition of any one of (1-1) to (1-6), further satisfying the following requirement (e):

(e) the composition comprises a mixture of quercetin glycosides in which n is 3, and in which other n values may be 1 or 2, or 1 and 2, in the total proportion of 70 mol% or more, quercetin glycosides in which n is 4 or more in the total proportion of 10 mol% or less, and a quercetin glycoside in which n is 0 in 20 mol% or less.

(1-8) The quercetin glycoside composition of any one of (1-1) to (1-7), further satisfying the following requirement (f):

(f) the composition comprises a mixture of 3 types of quercetin glycosides, one in which n is 1, one in which n is 2, and one in which n is 3.

(1-9) The quercetin glycoside composition of any one of (1-1) to (1-8), prepared by treating an enzymatically modified isoquercitrin with amylase.
(1-10) The quercetin glycoside composition of any one of (1-1) to (1-8), prepared by treating the enzymatically modified isoquercitrin with amylase and removing isoquercitrin therefrom, or by removing isoquercitrin from the enzymatically modified isoquercitrin and treating the remains with amylase.
(1-11) The quercetin glycoside composition of (1-9) or (1-10), wherein the amylase is β-amylase.

(2) Food Product

(2-1) A food product containing the quercetin glycoside composition of any one of (1-1) to (1-11).

(3) A method for preparing quercetin glycoside compositions having a high orally administered in vivo absorbability.

(3-1) A method for preparing the quercetin glycoside composition of (1-1) above having a higher orally administered in vivo absorbability than an enzymatically modified isoquercitrin, the method comprising a step of reducing a proportion of quercetin glycosides represented by the following formula:

[Formula 3]

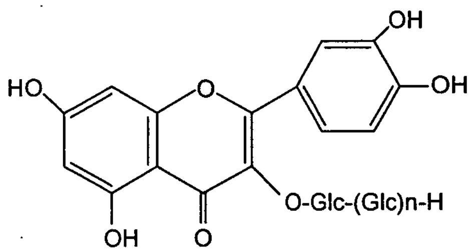

wherein Glc represents a glucose residue, and n is an integer of 4 or more, so as to make a total proportion thereof 15 mol% or less.
(3-2) The method of (3-1), wherein the step of reducing the proportion of quercetin glycosides represented by the formula includes treatment of the enzymatically modified isoquercitrin with amylase.
(3-3) The method of (3-2), wherein the amylase is β-amylase.
(3-4) The method of any one of (3-1) to (3-3), further comprising a step of reducing a proportion of isoquercitrin represented by the following formula:

[Formula 4]

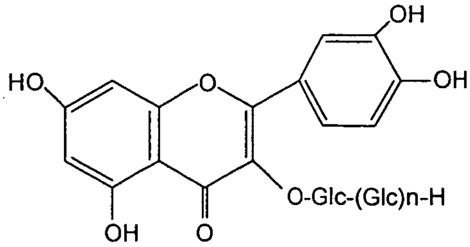

wherein Glc represents a glucose residue, and n is 0.

(3-5) The method for preparing the quercetin glycoside composition of (3-4), further comprising a step of removing isoquercitrin before and after the step of treating the enzymatically modified isoquercitrin with amylase.

(4) A Method for Enhancing orally administered in vivo absorbability

(4-1) A method for enhancing orally administered in vivo absorbability of quercetin glycoside compositions, comprising, using an enzymatically modified isoquercitrin as a starting material, a step of reducing a proportion of quercetin glycosides represented by the following formula:

[Formula 5]

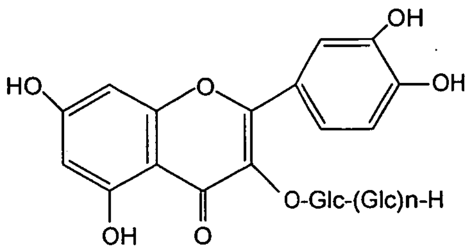

wherein Glc represents a glucose residue, and n is an integer of 4 or more.

(4-2) The method of (4-1), wherein the step of reducing the proportion of quercetin glycosides represented by the formula includes treatment of the enzymatically modified isoquercitrin with amylase.

(4-3) The method of (4-2), wherein the amylase is β-amylase.

(4-4) The method of any one of (4-1) to (4-3), further comprising a step of reducing a proportion of isoquercitrin represented by the following formula:

[Formula 6]

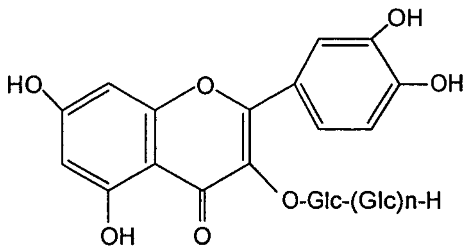

wherein Glc represents a glucose residue, and n is 0.

(4-5) The method of (4-4), further comprising a step of removing isoquercitrin before and after the step of treating the enzymatically modified isoquercitrin with amylase.

(4-6) A method for enhancing orally administered in vivo absorbability of quercetin glycoside compositions, comprising preparing a composition comprising a mixture of quercetin glycosides represented by the following formula:

[Formula 7]

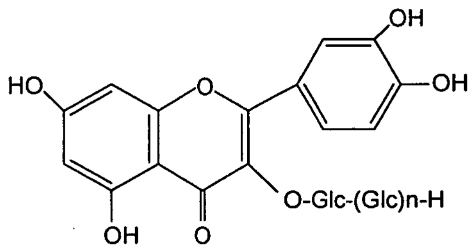

wherein Glc represents a glucose residue, and n is 0 or a positive integer of 1 or more, by treating an enzymatically modified isoquercitrin with amylase, the composition satisfying the following requirements (i) and (ii) :

(i) the composition comprises at least a quercetin glycoside in which n is 3, and
(ii) the composition comprises a mixture of quercetin glycosides in which n is 3, and in which other n values may be 1 or 2, or 1 and 2, in a total proportion of 50 mol% or more, and quercetin glycosides in which n is 4 or more in a total proportion of 15 mol% or less.

(4-7) The method of (4-6), further comprising preparing a quercetin glycoside composition satisfying the following requirement (iii):

(iii) the composition comprises a mixture of quercetin glycosides in which n is 3, and in which other n values may be 1 or 2, or 1 and 2, in the total proportion of 60 mol% or more, and a quercetin glycoside in which n is 0 in a proportion of 20 mol% or less.

(4-8) The method of (4-6) or (4-7), further comprising a step of preparing a quercetin glycoside composition satisfying the following requirement (iv):

(iv) the composition comprises a mixture of quercetin glycosides in which n is 3, and in which other n values may be 1 or 2, or 1 and 2, in the total proportion of 70 mol% or more, quercetin glycosides in which n is 4 or more in the total proportion of 10 mol% or less, and a quercetin glycoside in which n is 0 in 20 mol% or less.

(4-9) The method of any one of (4-6) to (4-8), further comprising a step of preparing a quercetin glycoside composition satisfying the following requirement (v):

(v) the composition comprises a mixture of 2 types of quercetin glycosides, one in which n is 2, and one in which n is 3, and the total proportion thereof is 50 mol% or more.

BRIEF DESCRIPTION OF THE DRAWINGS

Figure 1 shows the proportions of the components (of various quercitrin glycoside compositions (molar ratios (%) of IQC and IQC glycosides (IQC-G1, IQC-G2, IQC-G3, IQC-G4, IQC-G5, and IQC-G6)) obtained by β-amylase treatment of enzymatically modified isoquercitrin (Preparation Example 6).
Figure 2 shows experimental results indicating the orally administered in vivo absorbability of the G0 fraction, G1 fraction, G2 fraction, G3 fraction, and G4 fraction obtained in Preparation Example 1 (Experiment 1). The figure specifically shows AUC (Area under the curve) (0 to 3 hr) (μg/ml·hr) calculated based on the areas under the curves of the plasma concentration of quercetin-glucuronide conjugate and that of quercetin in rats to which these fractions have been orally administered.
Figure 3 shows experimental results indicating the orally administered in vivo absorbability of enzymatically modified isoquercitrin (IQC-G(mix)), isoquercitrin G(1-3) fraction (IQC-G(1-3) fraction), isoquercitrin G(3-6) fraction (IQC-G (3-6) fraction), and isoquercitrin (IQC) (Experiment 2). Figures 3a and 3b show time-dependent changes in the plasma concentration of quercetin-glucuronide conjugate and that of quercetin in rats to which these components have been orally administered.

Figure 4 shows AUC (Area under the curve) (0 to 3 hr) (μg/ml·hr) of IQC-G(mix), IQC-G (1-3) fraction, IQC-G(3-6) fraction, and IQC calculated based on the areas under the curves of the plasma concentration of quercetin-glucuronide conjugate and that of quercetin shown in Figure 3a and Figure 3b, respectively.

Figure 5 shows experimental results indicating the orally administered in vivo absorbability of the enzymatically modified isoquercitrin (IQC-G(mix)) and isoquercitrin G(4-6) fraction (IQC-G(4-6) fraction) obtained in Preparation Example 3 (Experiment 3). The figure specifically shows AUC (Area under the curve) (0 to 3 hrs) (μg/ml·hr) calculated based on the areas under the curves of the plasma concentration of quercetin-glucuronide conjugate and that of quercetin in rats to which these fractions have been orally administered.

Figure 6 shows the evaluation results of the antioxidant properties of the plasma of rats, to which the IQC-G(mix), IQC-G(1-3) fraction, IQC-G(3-6) fraction, and IQC shown in Figures 3 and 4, as well as carboxy methylcellulose (CMC) (as a control), have been orally administered, based on FRAP (Ferrous Reducing Activity of Plasma) of the plasma (Experiment 4).

Figure 7 shows experimental results indicating the orally administered in vivo absorbability of Sample 1 (IQC-G (mix)) and Samples 3 to 5 obtained in Preparation Example 4 (Experiment 5). The figure specifically shows AUC (Area under the curve) (0 to 3 hrs) (μg/ml·hr) calculated based on the areas under the curves of the plasma concentration of quercetin-glucuronide conjugate and that of quercetin in rats to which these fractions have been orally administered.

Figure 8 shows experimental results indicating the orally administered in vivo absorbability of Samples 6 to 9 obtained in Preparation Example 5 (Experiment 6). The figure specifically shows AUC (Area under the curve) (0 to 3 hrs) (μg/ml·hr) calculated based on the areas under the curves of the plasma concentration of quercetin-glucuronide conjugate and that of quercetin in rats to which these fractions have been orally administered.

Figure 9 shows experimental results indicating the orally administered in vivo absorbability of Sample 1 (IQC-G (mix)) and Sample 2 obtained in Preparation Example 4 (Experiment 7). The figure specifically shows AUC (Area under the curve) (0 to 3 hrs) (μg/ml·hr) calculated based on the areas under the curves of the plasma concentration of quercetin-glucuronide conjugate and that of quercetin in rats to which these fractions have been orally administered.

Figure 10 shows experimental results indicating the orally administered in vivo absorbability of Sample A (IQC-G (mix)) and Samples B to D obtained in Preparation Example 7 (Experiment 8). The figure specifically shows AUC (Area under the curve) (0 to 3 hrs) (μg/ml·hr) calculated based on the areas under the curves of the plasma concentration of quercetin-glucuronide conjugate and that of quercetin in rats to which these fractions have been orally administered.

## BEST MODE FOR CARRYING OUT THE INVENTION

### I. Explanation of Terms

#### (I-1) Quercetin Glycoside and Quercetin Glycoside Composition

"Quercetin glycoside" as used herein includes isoquercitrin (quercetin 3-O-β-D-glucopyranoside) with a glucose linked by a β-bond to the third position of quercetin represented by the following formula (4) (hereinafter sometimes referred to simply as "IQC"), and α-glycosyl isoquercitrin with about 1 to about 15 glucoses attached by an α-1,4 bond to a glucose residue of the IQC.

[Formula 8]

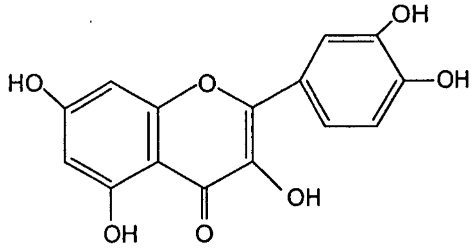

(4)

The above IQC and α-glycosyl isoquercitrin are both glycosides of quercetin, and thus collectively called "quercetin glycoside" in this specification without distinction between the two. Because α-glycosyl isoquercitrin is equivalent to a glycoside of IQC, it may also sometimes be referred to as "IQC glycoside" in this specification to make a distinction from IQC.

The quercetin glycoside composition to be attained by the present invention is a mixture of such IQC and various IQC glycosides at any desired ratio.

Specifically, the quercetin glycoside composition of the present invention is a mixture of quercetin glycosides represented by the following formula (1):

[Formula 9]

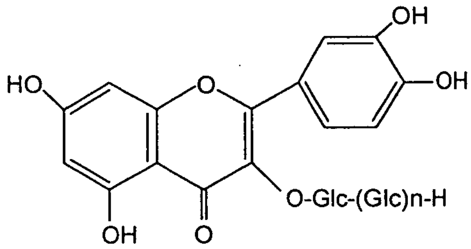

(1)

wherein Glc represents a glucose residue and n is 0 or a positive integer of 1 or more, the composition containing at least α-glycosyl isoquercitrin wherein n = 3.

In this specification, for the ease of explanation, among those represented by the above formula (1), IQC wherein n = 0 may be described as "G0" or "IQC-G0", the IQC glycoside wherein n = 1 (glycoside with one glucose residue linked to IQC by an α-1,4 bond) may be described as "G1" or "IQC-G1", the IQC glycoside wherein n = 2 (glycoside with two glucose residues linked to IQC by an α-1,4 bond) may be described as "G2" or "IQC-G2", the IQC glycoside wherein n = 3 (glycoside with three glucose residues linked to IQC by an α-1,4 bond) may be described as "G3" or "IQC-G3", the IQC glycoside wherein n = 4 (glycoside with four glucose residues linked to IQC by an α-1,4 bond) may be described as "G4" or "IQC-G4", the IQC glycoside wherein n = 5 (glycoside with five glucose residues linked to IQC by an α-1,4 bond) may be described as "G5" or "IQC-G5", the IQC glycoside wherein n = 6 (glycoside with six glucose residues linked to IQC by an α-1,4 bond) may be described as "G6" or "IQC-G6", ... and the IQC glycoside wherein n is m (glycoside with m glucose residues linked to IQC by an α-1,4 bond) may be described as "Gm" or "IQC-Gm" (m is an integer of 7 or more).

In this specification, the terms IQC, IQC glycoside, quercetin glycoside, G0, G1 (or "IQC-G1"), G2 (or "IQC-G2"), G3 (or "IQC-G3"), G4 (or "IQC-G4"), and ... Gm (or "IQC-Gm") are each used to mean a single compound or as a collective name for such compounds. When referring to a mixture comprising a combination of individual quercetin glycosides (G0, G1, G2, ... Gm), the term "quercetin glycoside composition" or "quercetin glycoside mixture" is used.

Further, in this specification, "total proportion of isoquercitrin G(1-3)" or "total proportion of IQC-G(1-3)" means the total proportion of quercetin glycosides (G1) wherein n is 1, quercetin glycosides (G2) wherein n is 2, and quercetin glycosides (G3) wherein n is 3 in the quercetin glycoside composition of the present invention. In this specification, "total proportion of isoquercitrin G(4≤)" or "total proportion of IQC-G(4≤)" means the total proportion of quercetin glycosides wherein n is 4 or more in the quercetin glycoside composition of the present invention.

(I-2) Enzymatically modified isoquercitrin

"Enzymatically modified isoquercitrin" as used herein is obtained by reacting a glucosyltransferase with IQC in the presence of a glycosyl donor (source of glucose) in accordance with a conventional method, and means a mixture of IQC and α-glycosyl isoquercitrin that has been glucosylated to various degrees (see, e.g., FFI Journal Vol. 209, No. 7, 2004, pp. 622-628; and Syokuhin Eisei Gaku Zasshi (Journal of Food Hygienics), Vol.41, No.1, pp. 54-60, etc.), represented by the following formula:

[Formula 10]

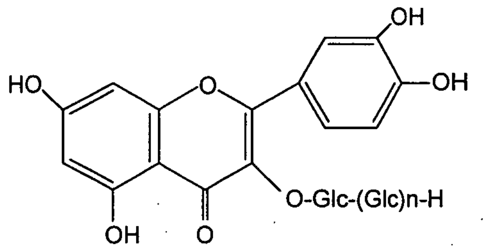

wherein Glc represents a glucose residue, and n is 0 or a positive integer of 1 or more.

Specifically, "enzymatically modified isoquercitrin" is a mixture of IQC of the above formula wherein the number of α-1,4-bonded glucose residues (n) is 0 and α-glycosyl isoquercitrin of the above formula wherein the number of α-1,4-bonded glucose residues (n) is 1 or more, usually 1 to 15, and preferably 1 to 10.

Examples of glucosyltransferases usable for IQC glycosylation processing include glucosidases such as α-amylase (E.C.3.2.1.1), α-glucosidase (E.C.3.2.1.20), etc.; and transglucosidases such as cyclodextrin glucanotransferase (E.C.2.4.1.19) (hereinafter referred to as CGTase), etc.

These glycosyltransferases are all commercially available enzymes. Examples of such commercial enzymatic agents include Contizyme (tradename) (product of Amano Enzyme Inc.). With respect to the amount of glycosyltransferase used, in the case of, for example, CGTase (having an enzyme specific activity of about 100 units, defining the amount of enzyme that generates 1 mg of β-cyclodextrin from soluble starch per minute as 1 unit), the glycosyltransferase may be used in an amount of 0.001 to 20 parts by weight per 1 part by weight of isoquercitrin. The amount is preferably about 0.005 to about 10 parts by weight, and more preferably about 0.01 to about 5 parts by weight.

As a glycosyl donor for glycosylation (source of glucose), any of those that allow at least one molecule of its glucose residue to be transferred to one molecule of IQC may be used. Examples thereof include glucose, maltose, amylose, amylopectin, starch, liquefied starch, saccharized starch, cyclodextrin, etc. The amount of glucose source used may be, per 1 part by weight of isoquercitrin present in the reaction system, usually 0.1 to 20 parts by weight, preferably 0.5 to 15 parts by weight, and more preferably 1 to 10 parts by weight.

"Enzymatically modified isoquercitrin" can be prepared, for example, although this depends on the kind of enzyme used, by reacting a glucosyltransferase with IQC in the presence of the above glycosyl donor (source of glucose) at 80 °C or less, preferably about 20 to about 80 °C, and more preferably about 40 to about 75 °C, usually at a pH of about 3 to about 11, and preferably at a pH of about 4 to about 8. The proportions of the components thereof are usually as follows.

[Table 1]

| Molar ratio (%) | | | | | | | | |
|---|---|---|---|---|---|---|---|---|
| Molar Ratio (%) | | | | | | | | |
| G0 | G1 | G2 | G3 | G4 | G5 | G6 | G7 | G8 or more |
| 23 ± 8 | 22 ± 3 | 24 ± 4 | 12 ± 2 | 8 ± 2 | 5 ± 2 | 3 ± 2 | 2 ± 1 | 1 ± 1 |

The above reaction may be performed in a static state, or while stirring or shaking. In order to prevent oxidation during the reaction, the headspace of the reaction system may be purged with nitrogen or a like inert gas. Ascorbic acid or like antioxidants may also be added to the reaction system.

In addition to the preparation from isoquercitrin as described above, enzymatically modified isoquercitrin may also be prepared using rutin as a starting material. In this case, after α-1,6-rhamnosidase (E.C.3.2.1.40) is reacted with rutin to produce isoquercitrin, enzymatically modified isoquercitrin can be prepared in accordance with the above method. ' Any α-1,6-rhamnosidases can be used insofar as it has an activity to produce isoquercitrin from rutin, and examples of commercial products thereof include hesperidinase and naringinase (products of Tanabe Seiyaku Co., Ltd.), and cellulase A "Amano" 3 (product of Amano Enzyme Inc.) .

II. Quercetin Glycoside Composition

The quercetin glycoside composition of the present invention is a mixture of quercetin glycosides represented

by Formula (1) below, and comprises at least α-glycosyl isoquercitrin in which n is 3:

[Formula 11]

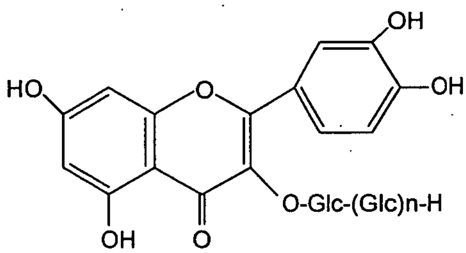

(1)

wherein Glc represents a glucose residue, and n is 0 or a positive integer of 1 or more.

More specifically, the quercetin glycoside composition of the present invention comprises α-glycosyl isoquercitrin in which n is 3 in Formula (1), and satisfies the following requirement (a):

(a) the composition comprises a mixture of quercetin glycosides in which n is 3, and in which other n values may be 1 or 2, or 1 and 2 (IQC-G (1-3)), in the total proportion of 50 mol% or more, and quercetin glycosides in which n is 4 or more (IQC-G (4≤)) in the total proportion of 15 mol% or less.

As shown in Experiments 1 to 3, 5, 7 and 8 to be described later, compositions containing a large amount of α-glycosyl isoquercitrin, wherein the number of glucose residues (n) bonding to IQC by α-1,4 bonding ranges from 1 to 3 (G1, G2, G3), have higher in vivo absorbability via oral administration (migration into the blood) than known enzymatically modified isoquercitrin; compositions containing a large amount of α-glycosyl isoquercitrin, wherein the number of glucoses (n) ranges from 4 to 6 (G4, G5, G6); and compositions containing a large amount of α-glycosyl isoquercitrin, wherein the number of glucose residues (n) ranges from 3 to 6 (G3, G4, G5, G6). For this reason, such compositions exhibit excellent in vivo antioxidative abilities when orally administered. Among α-glycosyl isoquercitrins, wherein the number of glucose residues (n) ranges from 1 to 3, particularly α-glycosyl isoquercitrin wherein the number of glucose residues (n) is 3 (G3), followed by α-glycosyl isoquercitrin wherein the number of glucose residues (n) is 2 (G2), have high in vivo absorbabilities (migration into the blood). In contrast, α-glycosyl isoquercitrin wherein the number of glucose residues (n) is 4 (G4) tends to have lower in vivo absorbability (migration into the blood) than α-glycosyl isoquercitrin wherein the number of glucose residues (n) is 3 (G3) (see Experiment 1 and Fig. 2).

Therefore, as described above, the quercetin glycoside composition of the present invention comprises G3, and contains IQC-G (4≤) in the total proportion of 15 mol% or less, and IQC-G (1-3) in the total proportion of 50 mol% or more, preferably 55 mol% or more, more preferably 60 mol% or more, further preferably 65 mol% or more, furthermore preferably 70 mol% or more, yet furthermore preferably 75 mol% or more, particularly preferably 80 mol% or more, and yet more particularly preferably 85 mol% or more, of the whole composition.

As mentioned above, the quercetin glycoside composition tends to have lower in vivo absorbability (migration into the blood) when α-glycosyl isoquercitrin wherein n is 4 or more (IQC-G(4≤)) is contained in a large proportion. For this reason, the proportion of IQC-G(4≤) contained in the quercetin glycoside composition of the present invention (total amount) is preferably even less than 15 mol%. For example, (IQC-G(4≤)) is contained in a proportion of 10 mol% or less, and preferably 6 mol% or less.

The quercetin glycoside composition of the present invention may contain isoquercitrin (IQC) wherein n is 0 (G0). However, the smaller the proportion of (IQC) (G0) is, the better because the total amount of α-glycosyl isoquercitrin wherein n ranges from 1 to 3 can be a larger proportion of the whole quercetin glycoside composition. The proportion of (IQC)(G0) contained in the quercetin glycoside composition of the present invention is, for example, 45 mol% or less, preferably 30 mol% or less, more preferably 20 mol% or less, and yet preferably 10 mol% or less.

The quercetin glycoside composition of the present invention preferably meets the following requirement (b), in addition to the above requirement (a):

(b) the proportion of a quercetin glycoside wherein n is 0 is 20 mol% or less of the composition.

Another preferable embodiment of the quercetin glycoside composition of the present invention meets the following requirement (c), in addition to the above requirement (a), or in addition to the above requirements (a) and (b):

(c) the composition comprises a mixture of two types of α-glycosyl isoquercitrins, one wherein n is 2, and one wherein n is 3 (G2 and G3), and the total proportion thereof (IQC-G(2-3)) is 50 mol% or more of the whole composition.

More preferably, the total proportion of IQC-G(2-3) includes 55 mol% or more, 60 mol% or more, 65 mol% or more, 70 mol% or more, and 75 mol% or more.
Yet another preferable embodiment of the quercetin glycoside composition of the present invention comprises G3, contains IQC-G(4≤) in the total proportion of 15 mol% or less, and meets the following requirement (d):

(d) the composition comprises a mixture of quercetin glycosides wherein n is 3, and wherein other n values may be 1 or 2, or 1 and 2, (IQC-G(1-3)) in the total proportion of 60 mol% or more, and a quercetin glycoside wherein n is 0 (IQC or G0) in a proportion of 20 mol% or less.

More preferably, quercetin glycoside compositions satisfying the above requirement (d) comprise a mixture of quercetin glycosides wherein n is 3, and wherein other n values may be 1 or 2, or 1 and 2, (IQC-G(1-3)) in the total proportion of 70 mol% or more, preferably 80 mol% or more, and more preferably 85 mol% or more. Further, quercetin glycosides wherein n are 4 or more are contained in the total proportion of 10 mol% or less, and preferably 6 mol% or less. Further preferably, a quercetin glycoside wherein n is 0 (IQC or G0) is contained in a proportion of 10 mol% or less.
Yet another embodiment of the quercetin glycoside composition of the present invention comprises α-glycosyl isoquercitrin wherein n is 3 (G3) in Formula (1), and satisfies the following requirement (e):

(e) the composition comprises a mixture of quercetin glycosides wherein n is 3, and wherein other n values may be 1 or 2, or 1 and 2, (IQC-G(1-3)) in the total proportion of 70 mol% or more, quercetin glycosides wherein n is 4 or more (IQC-G(4≤)) in the total proportion of 10 mol% or less, and a quercetin glycoside wherein n is 0 (IQC or G0) in a proportion of 20 mol% or less.

More preferably, quercetin glycoside compositions satisfying the above requirement (e) comprise IQC-G(1-3) in the total proportion of 75 mol% or more, preferably 80 mol% or more, and more preferably 85 mol% or more. Further preferably, IQC-G(4≤) is contained in the total proportion of 6 mol% or less. Furthermore preferably, a quercetin glycoside wherein n is 0 (IQC or G0) is contained in a proportion of 10 mol% or less.

III. Method for Preparing Quercetin Glycoside Composition

The quercetin glycoside composition having a high orally administered in vivo absorbability of the present invention can be prepared, using an enzymatically modified isoquercitrin as a starting material, via a step of reducing the proportion of quercetin glycosides (IQC-G(4≤)) represented by the following formula:

[Formula 12]

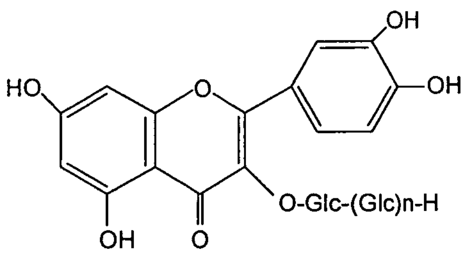

wherein Glc represents a glucose residue, and n is an integer of 4 or more, so as to make the total proportion of said quercetin glycosides 20 mol% or less.
The proportion of IQC-G (4≤) can be reduced using any method, and examples include a method in which fractions containing IQC-G(4≤) are removed from an enzymatically modified isoquercitrin, a method in which IQC-G(4≤) contained in an enzymatically modified isoquercitrin is decomposed, or the like. A preferable method is to treat an enzymatically modified isoquercitrin with amylase.
Amylases used herein may be enzymes having amylase activities, and the origins thereof are not limited. Examples include α-amylase (E.C.3.2.1.1), β-amylase (E.C.3.2.1.2), α-glucosidase (E.C.3.2.1.20), glucoamylase (E.C. 3.2.1.3), maltotriohydrolase, and like malto-oligosaccharide-producing enzymes.
β-Amylase is preferable. β-Amylase, when used as an amylase, can selectively reduce the proportion of α-glycosyl isoquercitrin wherein the number of α-1,4-bonding glucose residues (n) is 4 or more (IQC-G(4≤)) ; and increase the proportion of α-glycosyl isoquercitrin wherein n is 3, and wherein other n values may be 1 or 2, or 1 and 2, (IQC-G (1-3)) contained in the composition. Hence, the quercetin glycoside composition of the invention comprising G3, and meeting the following requirement (a) is readily prepared.

(a) The composition comprises IQC-G(1-3) in the total proportion of 50 mol% or more, and IQC-G (4≤) in the total proportion of 15 mol% or less.

The above-mentioned β-amylase, advantageously used in the present invention, is known to be contained in soybean, barley, wheat, *daikon* radish, sweet potato, *Aspergillus oryzae, Bacillus cereus, Bacillus polymyxa, Bacillus megaterium,* etc., and β-amylase from any of these origins can be freely used in the invention. β-Amylase is a commercially available enzyme, and examples include β-Amylase #1500 (product of Nagase ChemteX Corporation) and Biozyme M5 (product of Amano Enzyme Inc.) as soybean β-amylase; β-amylase L (product of Nagase ChemteX Corporation) and Biozyme/ML (product of Amano Enzyme Inc.) as barley β-amylase ; Biozyme M (product of Amano Enzyme Inc.) as whole-grain rice β-amylase ; and Uniase L (product of Yakult Pharmaceutical Industry Co., Ltd.) as *Aspergillus oryzae* β-amylase.

β-Amylase does not necessarily have to be purified, and may be purified crudely insofar as the object of the present invention can be achieved. For example, fractions containing β-amylase (e.g., extracts from soybean, barley, etc.) may be mixed with an enzymatically modified isoquercitrin and reacted. Alternatively, β-amylase is immobilized, and reacted batchwise or continuously with an enzymatically modified isoquercitrin.

Reaction conditions for β-amylase are not restricted so long as β-amylase reacts to an enzymatically modified isoquercitrin. Preferable conditions are those producing quercetin glycoside compositions which comprise G3, and contain IQC-G(4≤) in the total proportion of 15 mol% or less, and IQC-G(1-3) in the total proportion of 50 mol% or more, preferably 55 mol% or more, more preferably 60 mol% or more, further preferably 65 mol% or more, yet more preferably 70 mol% or more, yet further more preferably 75 mol% or more, particularly preferably 80 mol% or more, and yet particularly preferably 85 mol% or more, of the whole composition. Further conditions include those that produce quercetin glycoside compositions containing IQC-G (4≤) in the proportion (total amount) of 15 mol% or less, e.g., 10 mol% or less, and preferably 6 mol% or less.

When an enzyme of, for example, 4000U/g is used as a reaction condition for β-amylase, the amount of β-amylase to be used can be suitably selected from amounts ranging from 0.0001 to 0.5 parts by weight, per part by weight of an enzymatically modified isoquercitrin. Preferable ratios are about 0.0005 to about 0.4 parts by weight, and more preferably about 0.001 to about 0.3 parts by weight. The amount of the enzymatically modified isoquercitrin in the reaction system is not limited, but, for an efficient reaction, is desirably in a proportion of typically 0.1 to 20 % by weight, preferably 0.5 to 10 % by weight, and more preferably 1 to 10 % by weight, per 100 % by weight of the reaction system.

The reaction temperature can be in a range of about 80 °C or less, and can be suitably selected from this range. The industrially advantageous temperatures in this range are from about 20 to about 80 °C, and preferably about 40 to about 75 °C. The pH conditions typically range from about pH 3 to 11, and preferably from pH 4 to 8.

The reaction can be performed in a static state, or while stirring or shaking. To prevent oxidation during the reaction, the headspace of the reaction system may be replaced with an inert gas such as nitrogen, etc., or an antioxidant such as ascorbic acid, etc., may be added to the reaction system.

A step of further reducing the isoquercitrin of the reaction product obtained by the method above can be performed as necessary. Such a reduction method is not limited insofar as isoquercitrin (IQC) (G0) can be removed and separated from the reaction product obtained by the above method, and standard purification methods can be freely combined.

Examples include a method in which the above reaction product is adjusted to be acidic, and cooled to precipitate IQC(G0) for removal; various resin treatments (absorption method, ion exchange method, gel filtration, etc.); membrane treatments (ultrafiltration membrane treatment, reverse osmosis membrane treatment, ion exchange membrane treatment, zeta potential membrane treatment, etc.); electrodialysis; salt precipitation; acid precipitation; recrystalization; solvent fractionation; active carbon treatment; etc.

The removal step of IQC may be conducted on the reaction product after amylase treatment as described above; however, the removal step can be performed on, for example, an enzymatically modified isoquercitrin before amylase treatment. In particular, when β-amylase is used as an amylase, the IQC content remains substantially unchanged before and after amylase treatment. For this reason, the resulting reaction product is not much different whether IQC is first removed for reduction from an enzymatically modified isoquercitrin followed by treating such an isoquercitrin with β-amylase, or an enzymatically modified isoquercitrin is first treated with β-amylase followed by removal of IQC for reduction therefrom.

Since the thus obtained quercetin glycoside composition has reduced proportions of IQC-G (4≤) and IQC (G0), the IQC-G (1-3) proportion in the whole composition can consequently be even higher. Such quercetin glycoside compositions are desirably those containing 60 mol% or more, preferably 65 mol% or more, more preferably 70 mol% or more, yet more preferably 75 mol% or more, further more preferably 80 mol% or more, and particularly more preferably 85 mol% or more, of IQC-G(1-3) in the whole composition (100 mol%). Among these, more preferable quercetin glycoside compositions in light of in vivo absorbability are those containing 50 mol% or more, preferably 55 mol% or more, more preferably 60 mol% or more, yet more preferably 65 mol% or more, further more preferably 70 mol% or more, and particularly more preferably 75 mol% or more, of IQC-G(2-3) in the whole composition (100 mol%). Proportions of IQC-G(4≤) and IQC(G0) in such quercetin glycoside compositions can be determined in accordance with the above IQC-G (1-3) contents, and preferable examples typically include 10 mol% or less, and preferably 2 mol% or less, of IQC-G(4≤), and 20 mol% or less, and preferably 10 mol% or less, of IQC(G0).

IV. Use of Quercetin Glycoside Composition

As shown in the below Experiments, when orally administered, the quercetin glycoside composition of the present invention has higher in vivo absorbability (migration into the blood) (orally administered in vivo absorbability) than isoquercitrin and enzymatically modified isoquercitrin, and, as a result, exhibits an excellent antioxidant property in the body when orally administered.

Therefore, the present invention provides a food including an antioxidant containing the above quercetin glycoside composition of the present invention as an active ingredient, specifically an antioxidant for use in the living body (hereinafter also referred to as "in vivo antioxidant"); and the quercetin glycoside composition of the present invention. The food thus has an in vivo antioxidant function (active oxygen scavenging ability).

The in vivo antioxidant is not limited in form, and can be prepared in any desired form suitable for oral administration, such as powders, granules, tablets, capsule products, or like solid forms; solutions, suspensions, or like liquid forms; pastes or like semi-solid forms; etc.

The proportion of the quercetin glycoside composition mixed with the antioxidant is not limited, and may be suitably selected from a range of 0.01 to less than 100% by weight. The amount of antioxidant used is not limited insofar as antioxidant effects are exerted in the living body, and can be suitably selected within a range such that the antioxidant contains 1 mg to 30 g of quercetin glycoside composition in one dose for an adult weighing 60 kg.

The antioxidant can be prepared as a formulation in any desired manner by further mixing diluents, carriers, additives, or like components into the quercetin glycoside composition. Diluents and carriers usable herein are not limited insofar as the effect of the invention is not impaired. Examples thereof include sucrose, glucose, fructose, maltose, trehalose, lactose, oligosaccharide, dextrin, dextran, cyclodextrin, starch, starch syrup, isomerized liquid sugar, and like saccharides; ethanol, propylene glycol, glycerol, and like alcohols; sorbitol, mannitol, erythritol, lactitol, xylitol, maltitol, reduced palatinose, reduced amylolysis products, and like sugar alcohols; triacetin and like solvents; gum arabic, carrageenan, xanthan gum, guar gum, gellan gum, pectin, and like polysaccharides; and water. Examples of additives include chelating agents and like auxiliaries, flavorings, spice extracts, antiseptic agents, etc.

When the above formulation is prepared using such diluents, carriers, or additives, it is desirable in view of usability that the formulation contains the quercitrin glycoside composition in a proportion of 0.01 to 100% by weight, and preferably 0.1 to 50% by weight.

Examples of foods having an in vivo antioxidant function (active oxygen scavenging ability) include the quercetin glycoside composition of the present invention itself; formulations (e.g., powder, glanules, tablets, capsule products, solution, drink, etc.), such as supplements, prepared by adding the above diluents, carriers, or additives to the quercetin glycoside composition; and functional foods (including foods for specified health use and conditional foods for specified health use) obtained by adding the quercetin glycoside composition of the present invention to common foods as one component to thereby provide the foods with an in vivo antioxidant function (active oxygen scavenging ability). Such foods include those that contain the above quercetin glycoside composition of the present invention and have an in vivo antioxidant function (active oxygen scavenging ability), and that are provided with an indication that they are for use to prevent or suppress in vivo oxidation reactions or problems caused thereby.

In the case of a food having an antioxidant function (active oxygen scavenging ability), the proportion of the quercetin glycoside composition therein is not limited unless the antioxidant function is impaired, and, usually, may be suitably selected from a range of 0.001 to 100% by weight.

Examples of such foods include, but not limited to, frozen desserts such as ice cream, ice milk, lactice, sherbets (sorbets), ice candies, and the like; drinks such as milk beverages, lactic acid bacteria beverages, soft drinks (including those containing fruit juice), carbonated beverages, fruit juice drinks, vegetable juice drinks, vegetable/fruit beverages, sports drinks, powdered beverages; alcohols such as liqueurs; coffee beverages, red tea beverages, and other tea drinks; soups such as consommé soups, potage soups, and the like; desserts such as puddings (e.g., custard puddings, milk puddings, puddings containing fruit juice, and the like), jellies, babaloa, yogurt, and the like; gums such as chewing gum, bubble gum, and the like (stick gum and sugar-coated gum balls); chocolates such as coated chocolates (e.g., marble chocolates, and the like), flavored chocolates (e.g., strawberry chocolates, blueberry chocolates, melon chocolates, and the like), and the like; candies such as hard candies (including bonbons, butterballs, marbles, and the like), soft candies (including caramels, nougats, gummy candies, marshmallows, and the like), drops, taffy, and the like; baked confections such as hard biscuits, cookies, *okaki* (rice crackers), *sembei* (rice crackers), and the like; *tsukemono* (pickles) such as *asa-zuke, shoyu-zuke, shio-zuke, miso-zuke, kasu-zuke, koji-zuke, nuka-zuke, su-zuke, karashi-zuke, moromi-zuke, ume-zuke, fukujin-zuke, shiba-zuke, shoga-zuke, chosen-zuke,* and *umezu-zuke;* sauces such as separate dressings, oil-free dressings, ketchups, dips, and sauce; jams such as strawberry jam, blueberry jam, marmalade, apple jam, apricot jam, preserves, and the like; fruit wines such as red wines and the like; processed fruits such as cherries, apricots, apples, strawberries and peaches preserved in syrup, and the like; processed meats such as ham, sausage, roast pork, and the like; processed fish cakes such as fish ham, fish sausage, fish fillets, *kamaboko* (steamed fish paste), *chikuwa* (baked fish paste), *hanpen* (cake of pounded fish), *satsumaage* (fried fish paste), *datemaki* (fish omelet), whale bacon, and the like; dairy products such as cheese and the like; noodles such as *udon* (wheat noodles), *hiyamugi* (fine wheat noodles), *somen* (fine wheat noodles), *soba* (buckwheat noodles), Chinese noodles, spaghetti, macaroni, *bifun* (rice noodles), *harusame* (starch noodles), wontons, and the like; and delicatessens, fu (breadlike food made of wheat gluten), *denbu* (mashed and seasoned fish), and various other processed food products.

The intake of the food of the present invention is not limited insofar as it exhibits an anti-oxidization effect in the living body, and can be suitably selected, for example, within a range such that the food contains 1 mg to 30 g of quercetin glycoside in a portion for an adult weighing 60 kg.

V. Method for Improving Orally Administered In Vivo Absorbability of Quercitrin Glycoside Composition

The present invention provides a method for improving the orally administered in vivo absorbability of a quercitrin glycoside composition. According to the method of the present invention, with respect to quercitrin glycoside compositions known to have an antioxidant activity, the orally administered in vivo absorbability thereof can be improved beyond conventionally known enzymatically modified isoquercitrin. As a result, the in vivo antioxidant activity of a quercitrin glycoside composition can be improved.

The method of the present invention can be performed through a step of, using enzymatically modified isoquercitrin as a starting material, reducing the proportion of quercetin glycoside (IQC-G(4≤)) represented by the following formula:

[Formula 13]

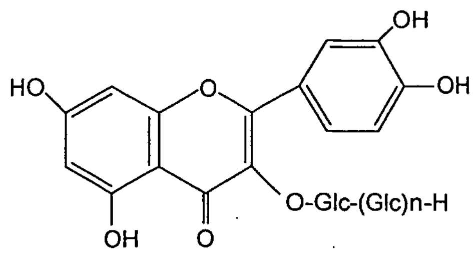

wherein Glc represents a glucose residue and n is an integer of 4 or more.

The method for reducing the proportion of IQC-G(4≤) is not limited, and methods that fractionate and remove IQC-G(4≤) from enzymatically modified isoquercitrin, methods that decompose IQC-G(4≤) contained in enzymatically modified isoquercitrin, and the like can be employed. A preferable example is one that processes enzymatically modified isoquercitrin with amylase, preferably β-amylase. The conditions for the reaction of amylase with enzymatically modified isoquercitrin may be the same as with the conditions given in III above.

The degree of reduction of IQC-G(4≤) may be such that the IQC-G(4≤) content (total proportion) in the final quercetin glycoside composition is 15 mol% or less, preferably 10 mol% or less, and more preferably 6 mol% or less.

To improve orally administered in vivo absorbability, the method may further contain a step of reducing the proportion of isoquercitrin (IQC or G0) represented by the following formula:

[Formula 14]

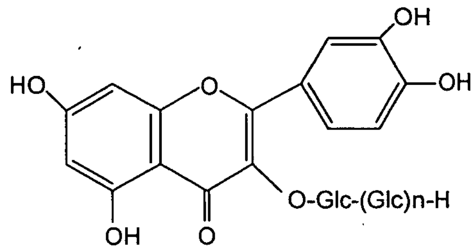

wherein Glc represents a glucose residue and n is 0.

Such a method is not limited insofar as isoquercitrin (IQC or G0) can be reduced, removed, or eliminated from the reaction product obtained by the above IQC-G(4≤) reduction processing, and may be a combination of any of the various conventional purification methods. Specific examples thereof are those described in III above.

The IQC reduction/removal step can be performed after the amylase treatment on the resulting reaction product as described above, and it may also be performed prior to the amylase treatment, for example, on enzymatically modified isoquercitrin.

The method of the present invention can be advantageously performed by subjecting enzymatically modified isoquercitrin to the above operation (amylase treatment or amylase treatment + removal and reduction of IQC), thereby converting the enzymatically modified isoquercitrin into the following quercetin glycoside composition: a composition comprising a mixture of quercetin glycosides represented by the following formula:

[Formula 15]

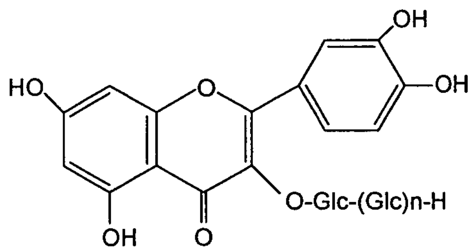

wherein Glc represents a glucose residue and n is 0 or a positive integer of 1 or more, and satisfying the following requirements (1) and (2):

(1) containing at least a quercetin glycoside wherein n is 3,
(2) the composition comprises a mixture of quercetin glycosides in which n is 3, and in which other n values may be 1 or 2, or 1 and 2, in a total proportion of 50 mol% or more, and quercetin glycosides in which n is 4 or more in a total proportion of 15 mol% or less.
Such a composition preferably further satisfies the following requirement (3):
(3) the composition comprises a mixture of quercetin glycosides in which n is 3, and in which other n values may be 1 or 2, or 1 and 2, in a total proportion of 60 mol% or more, and a quercetin glycoside in which n is 0 in a total proportion of 20 mol% or less.
Such a composition preferably further satisfies the following requirement (4):
(4) the composition comprises a mixture of quercetin glycosides in which n is 3, and in which other n values may be 1 or 2, or 1 and 2, in a total proportion of 70 mol% or more, quercetin glycosides in which n is 4 or more in a total proportion of 10 mol% or less, and a quercetin glycoside in which n is 0 in a total proportion of 20 mol% or less.
Such a composition preferably further satisfies the following requirement (5):
(5) the composition comprises a mixture of 2 types of quercetin glycosides, one in which n is 2, and one in which n is 3, and the total proportion thereof is 50 mol% or more.

According to this method, enzymatically modified isoquercitrin can be converted into a quercetin glycoside composition that exhibits, when orally administered, in vivo absorbability that is 1.3 times or more that of enzymatically modified isoquercitrin. The increase in vivo absorbability is preferably 1.01 to 5 times, and more preferably 1.01 to 2 times.

EFFECTS OF THE INVENTION

The quercetin glycoside compositions of the present invention have good in vivo absorbability when orally administered, compared to isoquercitrin and conventional enzymatically modified isoquercitrin. As a result, they exhibit high in vivo antioxidant effects. For this reason, the quercetin glycoside compositions and food products containing such compositions of the present invention are thought to be capable of eliminating reactive oxygen species in various parts of the body, preventing the formation of cytopathy and aging, thereby preventing, treating and ameliorating various diseases caused by reactive oxygen species.

EXAMPLES

The present invention will be described hereinafter with reference to Preparation Examples, Experiments, and Examples. However, the present invention is not limited thereto.

Reference Preparation Example 1: Preparation of Enzymatically modified isoquercitrin

(1) Preparation of Isoquercitrin

Two-hundred-fifty grams of flower buds of Japanese pagoda tree, a legume, was immersed in 2500 mL of hot water (95 °C or more) for two hours and then separated by filtration. The filtrate was obtained as a "first extract". The filtered residue was further immersed in hot water and extracted, giving a "second extract". These first and second extracts were combined and cooled to 30°C or less, and the precipitate formed by cooling was separated by filtration. The precipitate was washed with water, recrystallized, and dried, giving 22.8 g of rutin with a purity of 95% or more.

Subsequently, 20 g of this rutin was dispersed in 400 mL of water. The pH was adjusted to 4.9 using a pH adjuster, and 0.12 g of Naringinase (product of Amano Enzyme Inc., tradename "naringinase 'Amano' ", 3,000 U/g) was added thereto to start the reaction. The mixture was maintained at 72 °C for 24 hours. The reaction mixture was then cooled to 20 °C, and the precipitate produced by cooling was separated by filtration. The obtained precipitate (solid) was washed with water and then dried, and 13.4 g of isoquercitrin was collected.

(2) Preparation of Enzymatically modified isoquercitrin

To 10 g of the isoquercitrin obtained above was added 500 mL of water, and 40 g of cornstarch was added and dispersed therein. Subsequently, 15 g of cyclodextrin glucanotransferase (CGTase: Amano Enzyme Inc., tradename "Contizyme", 600 U/ml) was added thereto to start the reaction, and the mixture was maintained at pH 7.25 and 60°C for 24 hours. The obtained reaction mixture was cooled, and then loaded onto a column (Φ3.0 x 40 cm) filled with synthetic adsorbent, Diaion® HP-20 (product of Mitsubishi Chemical Co.). The adsorbent was washed with 1000 mL of water. Subsequently, 600 mL of 50% by volume ethanol aqueous solution was loaded onto the column. The obtained eluate was concentrated under reduced pressure, and then freeze-dried, giving 12.8 g of enzymatically modified isoquercitrin (hereinafter referred to as "isoquercitrin G(mix)" or "IQC-G(mix)"). The obtained isoquercitrin G(mix) was subjected to HPLC under the following conditions to fractionate the components, and the components were analyzed using a mass spectroscope (LC/MS/MS, Japan Water Corporation, Quattro Micro).
<HPLC conditions>
Column : Inertsil® ODS-2 Φ4.6 x 250 mm (product of GL Science Inc.)
Eluate : Water/acetonitrile/TFA = 850/15/2
Detection: Absorbance measurement at a wavelength of 351 nm
Flow rate: 0.8 mL/min

The results revealed that the above enzymatically modified isoquercitrin (IQC-G(mix)) comprised a mixture of IQC and various IQC glycosides represented by the following formula:

[Chemical formula 16]

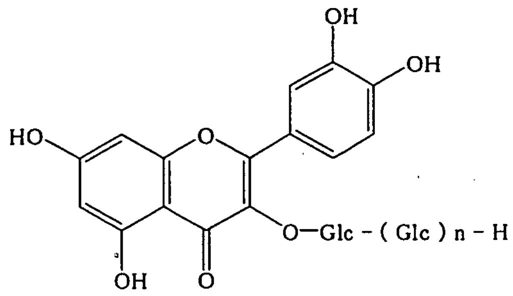

wherein Glc represents a glucose residue and n is 0 or a positive integer of 1 or more.

Molar ratios (%) of the IQC and IQC glycosides contained in the above mixture were calculated from the HPLC analysis results using the following equation. The proportions of the components were as shown in Table 2.

[Equation 1]

$$\text{The molar ratio of IQC or an IQC glycoside (\%)} = \frac{\text{the peak area of IQC or an IQC glycoside}}{\text{the total peak area of IQC and IQC glycosides}} \times 100$$

[Table 2]

| | Molar Ratio (%) | | | | | | | |
|---|---|---|---|---|---|---|---|---|
| | G0 isoquercitrin | G1 | G2 | G3 | G4 | G5 | G6 | G7 |
| IQC-G(mix) | 33 | 23 | 19 | 9 | 7 | 5 | 3 | 1 |

Preparation Example 1: Purification of IQC Glycoside (Gn)

The enzymatically modified isoquercitrin G(mix) (IQC-G(mix)) obtained in Reference Preparation Example 1 was subjected to HPLC under the following conditions, and then fractionated into a fraction containing abundant isoquercitrin (G0) (G0 fraction), a fraction containing abundant G1 (G1 fraction), a fraction containing abundant G2 (G2 fraction), a fraction containing abundant G3 (G3 fraction), and a fraction containing abundant G4 (G4 fraction).
<HPLC Fractionation Conditions>
Column : Develosil ODS-UG-15/30 or 5 cm x 50 cm
Solvent : Solvent A: aqueous solution containing 1% by volume acetic acid Solvent B: aqueous solution containing 1% by volume acetic acid and 90% by volume $CH_3CN$
Elution : Solvent B and solvent A are mixed at a ratio of 18% by volume to 82% by volume respectively, and eluted under isocratic conditions at a flow rate of 32 mL/min.
Detection: Absorbance detection at 360 nm

Specifically, an elution time from 40 minutes to 113 minutes was divided into 73 fractions taking 1 fraction per minute. Fractions 17 to 24, fractions 26 to 33, fractions 35 to 43, fractions 45 to 53, and fractions 55 to 73 were collected as a G4 fraction, G3 fraction, G2 fraction, G1 fraction, and G0 fraction, respectively. These fractions were freeze-dried, and about 300 mg of each was obtained as a solid.

Subsequently, the obtained G0 fraction, G1 fraction, G2 fraction, G3 fraction, and G4 fraction were each subjected to the HPLC analysis under the following conditions, and the molar ratios and the average molecular weights of the IQC and IQC glycosides contained in each fraction were calculated. The molar ratios (%) are shown in Table 3. As shown in Table 3, the G0 fraction, G1 fraction, G2 fraction, G3 fraction, and G4 fraction contained IQC (G0), IQC glycosides G1, G2, G3, and G4, respectively, in a proportion of 73% or more.
<HPLC Analysis Conditions>

Column : Develosil C30-UG-5 (4.6 x 150 mm)
Solvent : Solvent A: Aqueous solution containing 0.05% by volume TFA
Solvent B: $CH_3CN$ containg 0.05% by volume TFA
Elution : Gradient elution of solvent B 10% by volume → 80% by volume (0 to 20 min), solvent B 80% by volume → 80% by volume (20 to 25 min), solvent B 80% by volume→ 10% by volume (25 to 25.1 min), and solvent B 10% by volume (25.1 to 32 min)
Detection: Absorbance detection at a wavelength of 370 nm
Column temperature: 40 °C

[Table 3]

| | Molar Ratio (%) | | | | | | |
|---|---|---|---|---|---|---|---|
| Fraction sample | G0 isoquercitrin | G1 | G2 | G3 | G4 | G5 | G6 |
| G0 fraction | 73.5 | 15.8 | 6.7 | 1.9 | 1.1 | 0.7 | 0 |
| G1 fraction | 0 | 83.1 | 13.2 | 2.4 | 0.9 | 0.4 | 0 |
| G2 fraction | 0 | 0 | 88.9 | 9.2 | 1.9 | 0 | 0 |
| G3 fraction | 0 | 0 | 0 | 92.9 | 5.8 | 1.3 | 0 |
| G4 fraction | 0 | 0 | 0 | 0 | 90.2 | 9.0 | 0.9 |

Preparation Example 2: Preparation of Isoquercitrin G(1-3)

Fraction and Isoquercitrin G(3-6) Fraction

First, 0.65 g of the enzymatically modified isoquercitrin (IQC-G(mix)) obtained in Reference Preparation Example 1 was dissolved in aqueous methanol, and gel filtration chromatography was performed using a gel filtration resin (Sephadex® LH-20: Amersham Bioscience K K.). The filtrate was fractionated by a certain quantity, then subjected to HPLC analysis under the conditions described in the above Reference Preparation Example 1, and divided into the following two fractions: a fraction containing abundant G3, G4, G5, and G6 having three glucoses, four glucoses, five glucoses, and six glucoses, respectively, linked to IQC by an α-1,4 bond (hereinafter referred to as "isoquercitrin G(3-6) fraction" or an "IQC-G(3-6)" fraction); and a fraction containing abundant G1, G2, and G3 with one glucose, two glucoses, and three glucoses, respectively, linked to IQC by α-1,4 bond (hereinafter referred to as "isoquercitrin G(1-3) fraction" or "IQC-G(1-3) fraction"). Subsequently, these two fractions were concentrated under reduced pressure to remove solvent and then freeze-dried to give 0.15 g of "isoquercitrin G(3-6) fraction" ("IQC-G (3-6) fraction") and 0.1 g of "isoquercitrin G(1-3) fraction" ("IQC-G (1-3) fraction"). These fractions were subjected to HPLC analysis under the conditions described in the above Reference Preparation Example 1, and the molar ratios (%) of the IQC and IQC glycosides contained in each fraction were calculated.

The results are shown in Table 4. As shown in Table 4, the total proportion of G1, G2, and G3 contained in the IQC-G(1-3) fraction was 94%, and the total proportion of G3, G4, G5, and G6 contained in the IQC-G(3-6) fraction was 86%.

[Table 4]

| | Molar Ratio (%) | | | | | | | |
|---|---|---|---|---|---|---|---|---|
| | G0 isoquercitrin | G1 | G2 | G3 | G4 | G5 | G6 | G7 |
| IQC-G(1-3) fraction | 5 | 43 | 39 | 12 | 1 | 0 | 0 | 0 |
| IQC-G(3-6) fraction | 0 | 1 | 6 | 22 | 30 | 21 | 13 | 7 |

Preparation Example 3: Preparation of Enzymatically Modified Isoquercitrin G(mix) and Isoquercitrin G(4-6) Fraction

Enzymatically modified isoquercitrin was prepared in an identical manner as in Reference Preparation Example 1 (IQC-G(mix) (2)). The prepared IQC-G(mix)(2) was dissolved in aqueous methanol as in Preparation Example 2, and gel filtration chromatography and HPLC analysis were then performed to obtain a fraction containing abundant G4, G5, and G6, with four glucoses, five glucoses, and six glucoses, respectively, linked to IQC by an α-1,4 bond (hereinafter referred to as "isoquercitrin G(4-6) fraction" or an "IQC-G (4-6)" fraction). These fractions were each concentrated under

reduced pressure to remove solvent and then freeze-dried, giving 0.1 g of "isoquercitrin G(4-6) fraction" ("IQC-G(4-6)" fraction). The above IQC-G(mix) (2) and the "IQC-G(4-6)" fraction were subjected to HPLC analysis under the conditions described in the above Reference Preparation Example 1, and the molar ratios of the IQC and IQC glycosides were calculated.

The results are shown in Table 5. The total proportion of G4, G5, and G6 contained in the IQC-G(4-6) fraction was 83%.

[Table 5]

| | Molar Ratio (%) | | | | | | | |
|---|---|---|---|---|---|---|---|---|
| | G0 isoquercitrin | G1 | G2 | G3 | G4 | G5 | G6 | G7 |
| IQC-G(mix) (2) | 29 | 23 | 21 | 10 | 8 | 5 | 3 | 1 |
| IQC-G(4-6) fraction | 0 | 0 | 0 | 3 | 23 | 33 | 27 | 14 |

<u>Preparation Example 4:</u> Preparation of Samples 1 to 5

(1) Preparation of Sample 1

Following the procedures of Reference Preparation Examples 1 (1) and (2), enzymatically modified isoquercitrin (IQC-G(mix)) was prepared (Sample 1:IQC-G(mix) (3)).

(2) Preparation of Sample 2

Sample 1 (0.5 g) was dissolved in 50 mL of ion-exchange water, cooled with stirring and filtered, and then filtrate was passed through a column packed with synthetic adsorbent (product of Mitsubishi Chemical Co., Diaion® SP-207). The synthetic adsorbent was fully washed with water to remove unreacted glucoses and like impurities, then a 60% by volume ethyl alcohol aqueous solution was passed through the column, and the eluate was collected. The collected eluate was concentrated under reduced pressure, and then freeze-dried (Sample 2).

(3) Preparation of Sample 3

Sample 1 and isoquercitrin (IQC) were independently dissolved in methanol, and mixed so that the molar ratio of IQC was about 45%. Subsequently, this fraction was concentrated under reduced pressure to remove solvent, and then freeze-dried (Sample 3).

(4) Preparation of Sample 4

Sample 3 (0.5 g) was dissolved in 50 mL of ion-exchange water, then 2.5 mg of β-amylase (product of Amano Enzyme Inc., tradename "Biozyme M", 4000 U/g) was added thereto, and the mixture was maintained at 50 °C and pH 5.0 for 1.5 hours. After the enzyme was deactivated by heat treatment, the mixture was passed through a column packed with synthetic adsorbent (product of Mitsubishi Chemical Co., Diaion® SP-207). The synthetic adsorbent was fully washed with water to remove unreacted glucoses and like impurities, then a 60% by volume ethyl alcohol aqueous solution was passed through the column, and the eluate was collected. The collected eluate was concentrated under reduced pressure and then freeze-dried, giving a sample weighing 0.3 g (Sample 4) .

(5) Preparation of Sample 5

Sample 1 (5 g) was dissolved in aqueous methanol, and gel filtration chromatography was performed using a gel filtration resin (Sephadex® LH-20: Amersham Bioscience K.K.). The filtrate was fractionated by a certain quantity, and then subjected to HPLC analysis under the conditions described in the above Reference Preparation Example 1 to collect a fraction containing abundant G4, G5, G6, and G7. Subsequently, this fraction was then concentrated under reduced pressure to remove solvent and then freeze-dried. Subsequently, 1.0 g thereof was dissolved in 50 mL of ion-exchange water, then 7 mg of β-amylase (product of Amano Enzyme Inc., tradename "Biozyme M", 4000 U/g) was added thereto, and the mixture was maintained at 50 °C and pH 5.0 for 2 hours. After the enzyme was deactivated by heat treatment, the mixture was passed through a column packed with synthetic adsorbent (product of Mitsubishi Chemical Co., Diaion® SP-207). The adsorbent was fully washed to remove unreacted glucoses and like impurities, then a 60%

by volume ethyl alcohol aqueous solution was passed through the column, and the eluate was collected. The collected eluate was concentrated under reduced pressure and then freeze-dried (Sample 5, 0.2 g).

The above Samples 1 to 5 were subjected to HPLC under the conditions described in the above Reference Preparation Example 1, and the molar ratios (%) of the IQC and IQC glycosides contained in the samples were calculated. The proportions of the components were as follows. [Table 6]

| | Molar Ratio (%) | | | | | | | |
|---|---|---|---|---|---|---|---|---|
| | G0 | G1 | G2 | G3 | G4 | G5 | G6 | G7 |
| Sample 1 IQC-G(mix) (3) | 28.8 | 22.7 | 21.4 | 10.6 | 7.5 | 4.9 | 2.8 | 1.3 |
| Sample 2 | 15.4 | 23.9 | 26.0 | 13.4 | 9.7 | 6.4 | 3.6 | 1.7 |
| Sample 3 | 45.1 | 20.9 | 16.3 | 7.8 | 4.6 | 2.9 | 1.7 | 0.7 |
| Sample 4 | 42.9 | 25.8 | 20.6 | 9.3 | 0.5 | 0.3 | 0.5 | 0 |
| Sample 5 | 7.5 | 14.2 | 41.9 | 31.5 | 2.6 | 2.4 | 0 | 0 |

Preparation Example 5: Preparation of Samples 6 to 9

(1) Preparation of Sample 6

Following the procedures of Reference Preparation Examples 1 (1) and (2), enzymatically modified isoquercitrin (IQC-G(mix)) was prepared. This IQC-G (mix) and isoquercitrin (IQC) were independently dissolved in methanol, and mixed so that the molar ratio of IQC was about 45%. Subsequently, this fraction was concentrated under reduced pressure to remove solvent and then freeze-dried (Sample 6).

(2) Preparation of Samples 7, 8, and 9

In the same manner as in Preparation Example 1, Sample 1 was subjected to HPLC, and a fraction containing abundant G1 (G1 fraction), a fraction containing abundant G2 (G2 fraction), and a fraction containing abundant G3 (G3 fraction) were collected. These three fractions were independently dissolved in methanol, and each mixed with a methanol solution of Sample 6 so that the total proportions of IQC-G(1-3) therein were about 54%, 64%, and 80%, respectively. Subsequently, these fractions were concentrated under reduced pressure to remove solvent and then freeze-dried (Samples 7, 8, and 9).

The above Samples 6 to 9 were subjected to HPLC under the conditions described in the above Reference Preparation Example 1, and the molar ratios (%) of the IQC and IQC glycosides contained in the samples were calculated. The proportions of the components were as follows. [Table 7]

| | Molar Ratio (%) | | | | | | | |
|---|---|---|---|---|---|---|---|---|
| | G0 | G1 | G2 | G3 | G4 | G5 | G6 | G7 |
| Sample 6 | 45.1 | 20.9 | 16.3 | 7.8 | 4.6 | 2.9 | 1.7 | 0.7 |
| Sample 7 | 37.0 | 24.5 | 20.1 | 9.8 | 3.9 | 2.5 | 1.6 | 0.6 |
| Sample 8 | 24.9 | 23.7 | 27.2 | 13.3 | 4.6 | 3.2 | 1.9 | 1.2 |
| Sample 9 | 16.5 | 24.5 | 37.7 | 17.3 | 0.9 | 1.2 | 0.8 | 1.1 |

Reference Preparation Example 2: Preparation of Enzymatically Modified Isoquercitrin

(1) Preparation of Isoquercitrin

First, 5 kg of rutin was dispersed in 100 L of water, and the pH was adjusted to 4.9 using a pH adjuster. Subsequently, 30 g of Naringinase (Amano Enzyme Inc., tradename "naringinase 'Amano'", 3,000 U/g) was added thereto to start the reaction, and the mixture was maintained at 72 °C for 24 hours. The reaction mixture was then cooled to 30°C, and the precipitate obtained by cooling was separated by filtration. The obtained solid was washed and then dried to collect isoquercitrin.

(2) Preparation of Enzymatically Modified Isoquercitrin

To 2 kg of the obtained isoquercitrin was added 100 L of water, and 8 kg of cornstarch was added and dispersed therein. Subsequently, 3 L of CGTase (Amano Enzyme Inc., tradename "Contizyme", 600 U/ml) was added thereto, and the mixture was maintained at 60 °C and pH 7.25 for 24 hours. This mixture was cooled and then filtered, giving enzymatically modified isoquercitrin (referred to as "isoquercitrin G(mix)" or "IQC-G(mix)") (liquid). This IQC-G(mix) was subjected to HPLC under the conditions described in the above Reference Preparation Example 1 and thus analyzed, and molar ratios (%) were calculated. The results revealed that it comprised a mixture of IQC and various IQC glycosides in the proportions shown in Table 8. HPLC analysis was performed to calculate molar ratios (%) of the IQC and IQC glycosides contained in IQC-G (mix). [Table 8]

| | Molar ratio (%) | | | | | | | | |
|---|---|---|---|---|---|---|---|---|---|
| | G0 | 21 | G2 | G3 | G4 | G5 | G6 | G7 | G8 |
| IQC-G(mix) (5) | 22 | G1 | 20 | 13 | 9 | 6 | 4 | 2 | 3 |

Preparation Example 6: Control of β-amylase treatment

Following the procedures of Reference Preparation Examples 2 (1) and (2), enzymatically modified isoquercitrin (reaction mixture) was prepared. To 50 L of this reaction mixture was added 4 g of β-amylase (product of Amano Enzyme Inc., tradename "Biozyme M", 4000 U/g). The mixture was then maintained at 50°C and pH 5.0 for a certain period of time, and a portion thereof was collected. After the enzyme was deactivated by heat treatment, the collected reaction mixture was passed through a column packed with synthetic adsorbent (prodouct of Mitsubishi Chemical Co., Diaion® SP-207). The adsorbent fully washed with water to remove unreacted glucoses and like impurities, then a 60% by volume ethyl alcohol aqueous solution was passed through the column, and the eluate was collected. The collected eluate was concentrated under reduced pressure and then freeze-dried, thereby giving various quercetin glycoside compositions prepared by reacting β-amylase over different periods of time. These compositions were subjected to HPLC under the conditions described in Reference Preparation Example 1, and the molar ratios (%) of the IQC and IQC glycosides were calculated.

The results are shown in Table 9 and Figure 1. The molar ratio of isoquercitrin (IQC) was almost constant regardless of the reaction time. As the reaction proceeded, the proportions of G1 with one glucose linked to IQC by an α-1,4 bond and G2 with two glucoses linked to IQC by an α-1,4 bond (IQC-G(1-2)) increased. Finally, a quercetin glycoside composition formed of G0, G1, and G2 was provided (not illustrated or described). With respect to G3 with three glucoses linked to IQC by an α 1,4-bond, at an early stage of the reaction, G(4≤) with four or more glucoses linked to IQC by an α-1,4 bond decomposed and became G3 in part, and the molar ratio of IQC-G3 thus increased until a certain point in the reaction. However, after G(4≤) disappeared (reaction time: about 60 min.), G3 subsequently started to decompose, and the molar ratio of IQC-G3 thus decreased gradually.

[Table 9]

| | Molar Ratio (%) | | | | | | |
|---|---|---|---|---|---|---|---|
| | G0 | G1 | G2 | G3 | G4 | G5 | G6 |
| Reaction Time 0 minutes | 20.0 | 24.9 | 23.3 | 12.7 | 9.1 | 6.1 | 3.9 |
| 15 | 19.8 | 25.9 | 29.3 | 15.4 | 4.5 | 2.9 | 2.2 |
| 30 | 19.8 | 27.3 | 32.4 | 15.7 | 2.1 | 1.3 | 1.4 |
| 60 | 20.0 | 30.5 | 35.2 | 14.3 | 0.0 | 0.0 | 0.0 |
| 90 | 19.9 | 32.4 | 35.4 | 12.3 | 0.0 | 0.0 | 0.0 |
| 120 | 19.8 | 33.7 | 35.5 | 10.9 | 0.0 | 0.0 | 0.0 |
| 180 | 19.8 | 35.4 | 35.5 | 9.3 | 0.0 | 0.0 | 0.0 |
| 240 | 19.7 | 36.2 | 35.6 | 8.5 | 0.0 | 0.0 | 0.0 |
| 300 | 19.6 | 36.7 | 35.6 | 8.0 | 0.0 | 0.0 | 0.0 |
| 360 | 19.6 | 37.1 | 35.6 | 7.7 | 0.0 | 0.0 | 0.0 |

(continued)

| | Molar Ratio (%) | | | | | | |
|---|---|---|---|---|---|---|---|
| | G0 | G1 | G2 | G3 | G4 | G5 | G6 |
| 420 | 19.6 | 37.3 | 35.6 | 7.5 | 0.0 | 0.0 | 0.0 |

Preparation Example 7: Preparation of Samples A to D

(1) Preparation of Sample A

Enzymatically modified isoquercitrin prepared following the procedures of Reference Preparation Examples 2 (1) and (2) was passed through a column packed with synthetic adsorbent (product of Mitsubishi Chemical Co., Diaion® SP-207). The adsorbent was fully washed with water to remove unreacted glucoses and like impurities, then a 60% by volume ethyl alcohol aqueous solution was passed through the column, and the eluate was collected. The collected eluate was concentrated under reduced pressure, then dried, and ground into powder (Sample A: IQC-G (mix)) .

(2) Preparation of Sample B

Sample A (reaction mixture) was cooled with stirring and filtered, and then filtrate was passed through a column packed with synthetic adsorbent (product of Mitsubishi Chemical Co., Diaion® SP-207). The adsorbent was fully washed with water to remove unreacted glucoses and like impurities. A 60% by volume ethyl alcohol aqueous solution was then passed through the column, and the eluate was collected. The collected eluate was concentrated under reduced pressure and then freeze-dried, giving Sample B.

(3) Preparation of Samples C and D

First, 50 L of Sample A (reaction mixture) was cooled with stirring and filtered, and 4 g of β-amylase (product of Amano Enzyme Inc., tradename "Biozyme M", 4000 U/g) was added to the obtained filtrate. The mixture was maintained at 50°C and pH 5.0 for 30 minutes, and another batch of the same mixture was maintained at 50°C and pH 5.0 for 420 minutes. After the enzyme was deactivated by heat treatment, the mixtures were independently passed through a column packed with synthetic adsorbent (product of Mitsubishi Chemical Co., Diaion® SP-207). Each of the adsorbent was fully washed to remove unreacted glucoses and like impurities. A 60% by volume ethyl alcohol aqueous solution was then passed through each column, and the eluate was collected. The collected eluates were concentrated under reduced pressure and then freeze-dried, giving Sample C (β-amylase treatment for 30 minutes) and Sample D (β-amylase treatment for 420 minutes).

Samples A to D were subjected to HPLC under the conditions described in Reference Preparation Example 1, and the molar ratios (%) of the IQC and IQC glycosides contained in the samples were calculated. The proportions of the components were as shown in Table 10.

[Table 10]

| | Molar Ratio (%) | | | | | | | |
|---|---|---|---|---|---|---|---|---|
| | G0 | G1 | G2 | G3 | G4 | G5 | G6 | G7 |
| Sample A | 30.1 | 22.9 | 20.5 | 10.2 | 7.3 | 4.8 | 2.9 | 1.3 |
| Sample B | 17.1 | 21.3 | 22.8 | 13.2 | 10.7 | 7.7 | 4.9 | 2.3 |
| Sample C | 16.5 | 24.4 | 34.3 | 17.0 | 2.4 | 3.7 | 1.8 | 0.0 |
| Sample D | 16.9 | 44.4 | 38.2 | 0.5 | 0.0 | 0.0 | 0.0 | 0.0 |

Preparation Example 8

To 50 L of the IQC-G(mix) (5) prepared in Reference Preparation Example 2 was added 15 g of β-amylase (product of Amano Enzyme Inc., tradename "Biozyme M", 4000 U/g). The mixture was maintained at 50°C and pH 5.0 for 3 hours, and then the enzyme was deactivated by heat treatment. This reaction mixture (β-amylase-treated IQC-G (mix) (1)) was subjected to HPLC under the conditions described in the above Reference Preparation Example 1 and thus analyzed, and molar ratios (%) were calculated. The results revealed that it comprised a mixture of IQC and various IQC glycosides in the following proportions.

[Table 11]

| | Molar Ratio (%) | | | | |
|---|---|---|---|---|---|
| | G0 | G1 | G2 | G3 | G4 or more |
| β-Amylase-treated IQC-G (mix) (1) | 23 | 40 | 32 | 3 | 2 |

Preparation Example 9

To 50 L of the IQC-G (mix) (5) prepared in Reference Preparation Example 2 was added 4 g of β-amylase (product of Amano Enzyme Inc., tradename "Biozyme M", 4000 U/g). The mixture was maintained at 50°C and pH 5.0 for 1 hour, and then the enzyme was deactivated by heat treatment. This reaction mixture (β-amylase-treated IQC-G (mix) (2)) was subjected to HPLC under the above conditions and thus analyzed, and molar ratios (%) were calculated. The results revealed that it comprised a mixture of IQC and various IQC glycosides in the following proportions.

[Table 12]

| | Molar Ratio (%) | | | | |
|---|---|---|---|---|---|
| | G0 | G1 | G2 | G3 | G4 or more |
| β-Amylase-treated IQC-G(mix) (2) | 22 | 24 | 29 | 19 | 6 |

Preparation Example 10

To 50 L of the IQC-G (mix) (5) prepared in Reference Preparation Example 2 was added 15 g of β-amylase (product of Amano Enzyme Inc., tradename "Biozyme M", 4000 U/g). The mixture was maintained at 50°C and pH 5.0 for 3 hours, and then the enzyme was deactivated by heat treatment. This reaction mixture was cooled with stirring and filtered, and then loaded onto a column packed with synthetic adsorbent (product of Mitsubishi Chemical Co., Diaion® SP-207). The adsorbent was fully washed with water. A 60% by volume ethyl alcohol aqueous solution was then passed through the column, and the eluate was collected. The eluate (β-amylase-treated IQC-G(mix) (3)) was subjected to HPLC under the above conditions and thus analyzed, and molar ratios (%) were calculated. The results revealed that it comprised a mixture of IQC and various IQC glycosides in the following proportions.

[Table 13]

| | Molar Ratio (%) | | | | |
|---|---|---|---|---|---|
| | G0 | G1 | G2 | G3 | G4 or more |
| β-Amylase-treated IQC-G(mix) (3) | 14 | 20 | 64 | 1 | 1 |

Preparation Example 11

To 50 L of the IQC-G(mix) (5) obtained in Reference Preparation Example 2 was added 4 g of β-amylase (product of Amano Enzyme Inc., tradename "Biozyme M", 4000 U/g). The mixture was maintained at 50°C and pH 5.0 for 1 hour, and then the enzyme was deactivated by heat treatment. This reaction mixture was cooled with stirring and filtered, and then loaded onto a column packed with synthetic adsorbent (product of Mitsubishi Chemical Co., Diaion® SP-207). This adsorbent was fully washed with water, then a 60% by volume ethyl alcohol aqueous solution was passed through the column, and the eluate was collected. The eluate (β-amylase-treated IQC-G(mix) (4)) was subjected to HPLC under the above conditions and thus analyzed, and molar ratios (%) were calculated. The results revealed that it comprised a mixture of IQC and various IQC glycosides in the following proportions.

[Table 14]

| | Molar Ratio (%) | | | | |
|---|---|---|---|---|---|
| | G0 | G1 | G2 | G3 | G4 or more |
| β-Amylase-treated IQC-G(mix) (4) | 12 | 24 | 43 | 20 | 1 |

Preparation Example 12

To 50 L of the IQC-G (mix) (5) prepared in Reference Preparation Example 2 was added 15 g of β-amylase (product of Amano Enzyme Inc., tradename "Biozyme M", 4000 U/g). The mixture was maintained at 50°C and pH 5.0 for 3 hours, and then the enzyme was deactivated by heat treatment. This reaction mixture was cooled with stirring and filtered. The obtained filtrate was concentrated with a UF membrane having a molecular cutoff of 10000 (product of Asahi Kasei Chemicals Corporation, SEP-3053). The membrane permeate was collected and further concentrated with a UF membrane having a molecular cutoff of 1000 (product of Nihon Pall Ltd., Pall Filtron ultrafiltration membrane, Nova series) to prepare a concentrate. The concentrate was diluted with water to make 50 L, and then loaded onto a column packed with synthetic adsorbent (product of Mitsubishi Chemical Co., Diaion® SP-207). This adsorbent was fully washed with water. A 60% by volume ethyl alcohol aqueous solution was passed through the column, and the eluate was collected. This eluate (β-amylase-treated IQC-G(mix) (5)) was subjected to HPLC under the above conditions and thus analyzed, and molar ratios (%) were calculated. The results revealed that it comprised a mixture of IQC and various IQC glycosides in the following proportions.

[Table 15]

| | Molar Ratio (%) | | | | |
|---|---|---|---|---|---|
| | G0 | G1 | G2 | G3 | G4 or more |
| β-Amylase-treated IQC-G(mix) (5) | 14 | 19 | 65 | 1 | 1 |

Preparation Example 13

To 50 l of the IQC-G(mix) (5) prepared in Reference Preparation Example 2 was added 4 g of β-amylase (product of Amano Enzyme Inc., tradename "Biozyme M", 4000 U/g). The mixture was maintained at 50°C and pH 5.0 for 1 hour, and then the enzyme was deactivated by heat treatment. This reaction mixture was cooled with stirring, and then filtered. The obtained filtrate was concentrated with a UF membrane having a molecular cutoff of 10000 (product of Asahi Kasei Chemicals Corporation, SEP-3053) and thereby membrane-treated. The membrane permeate was thus collected and further concentrated with a UF membrane having a molecular cutoff of 1000 (product of Nihon Pall Ltd., Pall Filtron ultrafiltration membrane, Nova series) to prepare a concentrate. The concentrate was diluted with water to make 50 L, and then loaded onto a column packed with synthetic adsorbent (product of Mitsubishi Chemical Co., Diaion® HP-20). This adsorbent was fully washed with water, then a 60% by volume ethyl alcohol aqueous solution was passed through the column, and the eluate was collected. This eluate (β-amylase-treated IQC-G(mix) (6)) was subjected to HPLC under the above conditions and thus analyzed, and molar ratios (%) were calculated. The results revealed that it comprised a mixture of IQC and various IQC glycosides in the following proportions.

[Table 16]

| | Molar Ratio (%) | | | | |
|---|---|---|---|---|---|
| | G0 | G1 | G2 | G3 | G4 or more |
| β-Amylase-treated IQC-G (mix) (6) | 16 | 25 | 42 | 16 | 1 |

Preparation Example 14

To 50 L of the IQC-G(mix) (5) prepared in Reference Preparation Example 2 was added 1 g of β-amylase (product of Nagase ChemteX Corporation, β-amylase #1500, 15000 U/g). The mixture was maintained at 50°C and pH 5.0 for 3 hours, and then the enzyme was deactivated by heat treatment. This reaction mixture was cooled with stirring, and then filtered. The obtained filtrate was concentrated with a vacuum concentrator to a Brix value of 70. Eight times the amount of 95% by volume ethanol was added to this concentrate while stirring. The mixture was cooled to precipitate glucoses and like impurities, and the supernatant was then collected. This supernatant (β-amylase-treated IQC-G(mix) (7)) was subjected to HPLC under the above conditions and thus analyzed, and molar ratios (%) were calculated. The results revealed that it comprised a mixture of IQC and various IQC glycosides in the following proportions.

[Table 17]

| | Molar Ratio (%) | | | | |
|---|---|---|---|---|---|
| | G0 | G1 | G2 | G3 | G4 or more |
| β-Amylase-treated IQC-G (mix) (7) | 15 | 21 | 63 | 1 | 0 |

Preparation Example 15

To 50 L of the IQC-G(mix) (5) prepared in Reference Preparation Example 2 was added 5 g of β-amylase (product of Nagase ChemteX Corporation, β-amylase #1500, 15000 U/g). The mixture was maintained at 50°C and pH 5.0 for 1 hour, and then the enzyme was deactivated by heat treatment. This reaction mixture was cooled with stirring, and then filtered. The obtained filtrate was concentrated with a vacuum concentrator to a Brix value of 70. Eight times the amount of 95% by volume ethanol was added to this concentrate with stirring. The mixture was cooled to precipitate glucoses and like impurities, and the supernatant was then collected. This supernatant (β-amylase-treated IQC-G(mix) (8)) was subjected to HPLC under the above conditions and thus analyzed, and molar ratios (%) were calculated. The results revealed that it comprised a mixture of IQC and various IQC glycosides in the following proportions.

[Table 18]

| | Molar Ratio (%) | | | | |
|---|---|---|---|---|---|
| | G0 | G1 | G2 | G3 | G4 or more |
| β-Amylase-Treated IQC-G (mix) (8) | 16 | 24 | 43 | 17 | 0 |

EXPERIMENTS

Experiment 1: Measurement of Migration into the Blood (Orally Administered in Vivo Absorbability) (1)

The G0 fraction, G1 fraction, G2 fraction, G3 fraction, and G4 fraction prepared in Preparation Example 1 were examined for orally administered in vivo absorbability. Specifically, 30 SD male rats (7 to 9 weeks old) that had been fasted from the previous night were divided into five groups (six rats per group), and the G0 fraction, G1 fraction, G2 fraction, G3 fraction, and G4 fraction were orally administered to the five groups, respectively, at a dose of 150 μmol/kg of body weight. Blood was collected from the tail vein 0 minutes (before the administration), 30 minutes, 1 hour, and 3 hours after the administration, and heparin was added thereto. Centrifugation was then performed, and, from the supernatant, heparin plasma samples were prepared. The concentrations of quercetin-glucuronide conjugate and quercetin in the prepared heparin plasma samples were measured by HPLC under the following conditions. AUC (Area under the curve) (0 to 3 hr) (μg/ml·hr) was then calculated based on the area under the curve of the plasma concentration of quercetin-glucuronide conjugate (μg/ml) and the area under the curve of the plasma concentration of quercetin (μg/ml). Quercetin-glucuronide conjugate and quercetin are both in vivo metabolites of isoquercitrin. Therefore, in the following experiments, orally administered in vivo absorbability was evaluated based on the total of both AUC values.
<HPLC conditions>
Column : Develosil C30-UG-5 (4.6 x 150 mm)
Solvent : Solvent A: Aqueous solution containing 0.05% by volume TFA
Solvent B: $CH_3CN$ containing 0. 05% by volume TFA
Elution : Gradient elution of solvent B 10% by volume → 80% by volume (0 to 20 min), solvent B 80% by volume → 80% by volume (20 to 25 min), solvent B 80% by volume → 10% by volume (25 to 25.1 min), and solvent B 10% by volume (25.1 to 32 min)
Detection: Absorbance detection at a wavelength of 370 nm
Column temperature: 40 °C

The results are shown in Figure 2. As indicated by Figure 2, the orally administered in vivo absorbability (migration into the blood) was revealed to vary depending on the number of glucose residues linked to IQC by an α-1,4 bond. Specifically, as compared with the G0 fraction, from the G1 fraction to the G2 fraction, and then to the G3 fraction, the migration into the blood (orally administered in vivo absorbability) progressively increased with each increase in the number (n) of glucoses linked to IQC by an α-1,4 bond from 1 to 2 to 3. However, at a glucose number (n) of 4, migration into the blood (orally administered in vivo absorbability) decreased. This revealed that IQC-G3, IQC-G2, and IQC-G1 having three, two, and one glucoses linked to IQC, respectively, have higher absorbability in this order, and also that too small a number of glucoses (G0) as well as too large the number (G4 or more) reduce absorbability.

Experiment 2: Measurement of Migration into the Blood (Orally Administered In Vivo Absorbability) (2)

The isoquercitrin (IQC) and IQC-G(mix) prepared in Reference Preparation Example 1 and the IQC-G(3-6) fraction and-IQC-G(1-3) fraction prepared in Preparation Example 2 were examined for orally administered in vivo absorbability.

Specifically, 24 SD male rats (7 to 9 weeks old) that had been fasted from the previous night were divided into four groups (six rats per group). The IQC and IQC-G (mix) prepared in Reference Preparation Example 1 and the IQC-G(3-6) fraction and IQC-G(1-3) fraction prepared in Preparation Example 2 were orally administered respectively to the four groups, respectively, at a dose of 198 μmol/kg of body weight. Subsequently, in the same manner as in Experiment 1, plasma was prepared, and the plasma concentration of quercetin-glucuronide conjugate and that of quercetin were measured by HPLC. As a control, measurement was also performed for a group without administration.

The results are shown in Figure 3. Figure 3a shows time-dependent changes in the plasma concentration of quercetin-glucuronide conjugate (μg/ml) after the administration of the samples. Figure 3b shows time-dependent changes in the plasma concentration of quercetin (μg/ml) after the administration of the samples.

As indicated by Figure 3, when the IQC-G(1-3) fraction was orally administered, the serum concentration of quercetin-glucuronide conjugate and that of quercetin concentration both significantly increased in comparison not only with the case of IQC but also with the case of orally administering enzymatically modified isoquercitrin (IQC-G(mix)) or IQC-G(3-6) fraction.

Figure 4 shows AUC (Area under the curve) (0 to 3 hr) calculated based on the area under the curve of the plasma concentration of quercetin-glucuronide conjugate (μg/ml) shown in Figure 3a and the area under the curve of the plasma concentration of quercetin (μg/ml) shown in Figure 3b. Figure 4 reveals that the IQC-G(1-3) fraction had an AUC 1.4 to 1.5 times larger than those of the IQC-G(mix) and IQC-G(3-6) fraction.

Experiment 3: Measurement of Migration into the Blood (Orally Administered In Vivo Absorbability) (3)

The IQC-G (mix) (2) and IQC-G (4-6) fraction prepared in Reference Preparation Example 3 were examined for orally administered in vivo absorbability. Specifically, 12 SD male rats (7 to 9 weeks old) that had been fasted from the previous night were divided into two groups (six rats per group), and the isoquercitrin prepared in Reference Preparation Example 1 and the IQC-G(4-6) fraction prepared in Preparation Example 1 were orally administered to the two groups, respectively, at a dose of 198. μmol/kg of body weight. Subsequently, in the same manner as in Experiment 1, plasma was prepared, and the plasma concentration of quercetin-glucuronide conjugate and that of quercetin were measured by HPLC. AUC (Area under the curve) (0 to 3 hr) (μg/ml·hr) was then calculated based on the area under the curve of the plasma concentration of quercetin-glucuronide conjugate (μg/ml) and the area under the curve of the plasma concentration of quercetin (μg/ml).

The results are shown in Figure 5. As indicated by Figure 5, when the IQC-G (4-6) fraction was orally administered, the serum concentration of quercetin-glucuronide conjugate and the serum concentration of quercetin were lower than when orally administering enzymatically modified isoquercitrin (IQC-G(mix)).

The results shown in Figures 4 and 5 indicate that, as compared with when IQC-G(mix) was orally administered, oral administration of the IQC-G(4-6) fraction results in lower serum concentrations of quercetin-glucuronide conjugate and quercetin, while oral administration of the IQC-G(3-6) fraction results in equivalent. This suggests that IQC-G3 with three glucoses linked to IQC is responsible for in vivo absorption. The results also suggest that, when orally administered, quercetin glycoside compositions containing a small quantity of IQC-G(4-6) with 4 to 6 glucoses linked to IQC and/or quercetin glycoside compositions containing a large quantity of IQC-G(1-3) with 1 to 3 glucoses linked to IQC have enhanced migration into the blood (orally administered in vivo absorbability) as compared with conventional enzymatically modified isoquercitrin (IQC-G (mix)) .

Experiment 4: Measurement of Antioxidant Property

The plasma samples collected before administration (0 minutes), and also 30 minutes, 1 hour, and 3 hours after administration in the above Experiment 2 were examined for antioxidant property (FRAP: Ferrous Reducing Activity of Plasma), and the in vivo efficacy of oral administration of IQC, enzymatically modified isoquercitrin (IQC-G(mix)), an IQC-G(3-6) fraction, and an IQC-G(1-3) fraction was evaluated. FRAP was obtained by measuring the ability to reduce ferric iron to ferrous iron, according to the method of Iris et al. (Iris F F Benzie et al., Analytical Biochemistry 239, 70-76 (1996)).

Specifically, immediately after 40 μl of each subject plasma was independently added to 990 μl of a FRAP reagent (10 mM TPTZ (2,4,6-tri-(2-pyridyl)-s-triazine), 20 mM $FeCl_3 \cdot 6H_2O$ in 300 mM acetate buffer (pH 3.6)), the absorbance at 593 nm was monitored for 4 minutes. As a control test, 0.5% carboxymethylcellulose (CMC) instead of IQC or an IQC glycoside mixture was orally administered to rats, and the antioxidant property of the rat plasma was

measured. The FRAP activity (μmol/l) of each subject plasma was calculated based on the calibration curve formed using $FeSO_4$ (100 to 1000 μM) as a standard substance.

The results are shown in Figure 6. Taking the FRAP activity (μmol/l) value before oral administration of the samples (0 minutes) as 100%, the results show the FRAP activity after oral administration of the samples (30 minutes, 1 hour, and 3 hours) as a relative activity (%) thereto. As shown in Figure 6, with respect to plasma antioxidant property, it was confirmed that the IQC-G(1-3) fraction, which had been confirmed to have the highest in vivo absorbability in Experiments 1 and 2, had a significantly higher value as compared with the IQC-G(3-6) fractions, IQC-G(mix), and IQC.

Experiment 5: Measurement of Migration into the Blood (Orally Administered In Vivo Absorbability) (4)

The enzymatically modified isoquercitrin (Sample 1: IQC-G(mix) (3)), Sample 3, Sample 4, and Sample 5 obtained in Preparation Example 4 were examined for orally administered in vivo absorbability following the procedure of Experiment 1. Specifically, 24 SD male rats (7 to 9 weeks old) that had been fasted from the previous night were divided into four groups (six rats per group), and Samples 1, 3, 4, and 5 were orally administered to the four groups, respectively, at a dose of 198 μmol/kg of body weight. Subsequently, in the same manner as in Experiment 1, plasma was prepared, and the plasma concentration of quercetin-glucuronide conjugate and that of quercetin were measured by HPLC.

Figure 7 shows AUC (Area under the curve) (0 to 3 hr) (μg/ml·hr) calculated based on the area under the curve of the plasma concentration of quercetin-glucuronide conjugate (μg/ml) and the area under the curve of the plasma concentration of quercetin (μg/ml).

As indicated by these results, Sample 4 and Sample 5 containing IQC-G(1-3), including G3, in a total proportion of 55 mol% or more and IQC-G(4≤) in a total proportion of 5 mol% or less showed a significantly higher in vivo absorbability than conventional enzymatically modified isoquercitrin (Sample 1). Sample 5 which had a particularly high in vivo absorbability contained IQC-G (2- 3) in a total proportion of 50 mol% or more. With respect to Sample 3, although it contained G3 and the total proportion of IQC-G(4≤) therein was as small as 10 mol% or less, because the total proportion of IQC-G(1-3) was not more than 50 mol%, the in vivo absorbability was lower than conventional enzymatically modified isoquercitrin (Sample 1).

The comparison between the samples suggests the following.

(i) Sample 1 and Sample 3: An increase in the total proportion of isoquercitrin (IQC) reduces in vivo absorption.
(ii) Sample 3 and Sample 4: Reacting β-amylase with enzymatically modified isoquercitrin (IQC-G(mix)) increases in vivo absorption.
(iii) Sample 3, Sample 4, and Sample 5: Reacting β-amylase with IQC-G(mix) increases in vivo absorption. Reduction of isoquercitrin (IQC) further increases in vivo absorption.

Experiment 6: Measurement of Migration into the Blood (Orally Administered In Vivo Absorbability) (5)

Experiments as with the above Experiment 5 were performed for Sample 6, Sample 7, Sample 8, and Sample 9 obtained in Preparation Example 5. AUC (Area under the curve) (0 to 3 hr) (μg/ml·hr) was calculated based on the area under the curve of the plasma concentration of quercetin-glucuronide conjugate (μ/ml) and the area under the curve of the plasma concentration of quercetin (μg/ml).

The results are shown in Figure 8. In vivo absorbability for respective samples was found to be higher in the order of Sample 6 < Sample 7 < Sample 8 < Sample 9. These results suggest that the in vivo absorbability relates to the total proportion of IQC-G3 and the total proportion of IQC-G(1-3).

The total proportion of IQC-G3 and the total proportion of IQC-G(1-3) in each sample were as follows:
Sample 6 (G3: 7.8 mol%, G(1-3): 45.1 mol%), Sample 7 (G3: 9.8 mol%, G(1-3): 54.4 mol%), Sample 8 (G3: 13.3 mol%, G(1-3) : 64.2 mol%), and Sample 9 (G3: 17.3 mol%, G(1-3):79.6 mol%).

Experiment 7: Measurement of Migration into the Blood (Orally Administered In Vivo Absorbability) (6)

Experiments as with the above Experiment 5 were performed for the enzymatically modified isoquercitrin (Sample 1: IQC-G(mix) (3)) and Sample 2 obtained in Preparation Example 4. AUC (Area under the curve) (0 to 3 hr) (μg/ml·hr) was calculated based on the area under the curve of the plasma concentration of quercetin-glucuronide conjugate (μg/ml) and the area under the curve of the plasma concentration of quercetin (μg/ml). The results are shown in Figure 9. When Sample 2 that is a quercetin glycoside composition of Sample 1 with reduced IQC was orally administered, the in vivo absorbability was slightly higher than when orally administering of Sample 1 (IQC-G(mix)).

Experiment 8: Measurement of Migration into the Blood (Orally Administered In vivo Absorbability) (7)

The enzymatically modified isoquercitrin (Sample A (IQC-G(mix))) and Sample B, Sample C, and Sample D obtained in Preparation Example 7 were examined for orally administered in vivo absorbability following the procedure of Experiment 1. Specifically, 17 SD male rats (7 to 9 weeks old) that had been fasted from the previous night were divided into four groups (3 to 5 rats per group), and Samples A to D were orally administered to the four groups, respectively, at a dose of 198 μmol/kg of body weight. Subsequently, in the same manner as in Experiment 1, plasma was prepared, and the plasma concentration of quercetin-glucuronide conjugate and that of quercetin were measured by HPLC. Figure 10 shows AUC (Area under the curve) (0 to 3 hr) (μg/ml·hr) calculated based on the area under the curve of the plasma concentration of quercetin-glucuronide conjugate (μg/ml) and the area under the curve of the plasma concentration of quercetin (μg/ml).

As indicated by the results, Sample C and Sample D containing IQC-G(1-3), including G3, in a total proportion of 75 mol% or more and IQC-G(4≤) in a proportion of 10 mol% or less showed in vivo absorbability significantly higher than that of conventional enzymatically modified isoquercitrin (Sample A:IQC-G(mix)). Sample C which had particularly high in vivo absorbability contained IQC-G(2-3) in a total proportion of 50 mol% or more. With respect to Sample B, although the total proportion of IQC-G(1-3) including G3 was 50 mol% or more, the total proportion of IQC-G(4≤) was as relatively large as 26 mol%, and the in vivo absorbability was lower than that of conventional enzymatically modified isoquercitrin (Sample A).

Sample 1 and Sample 2 shown in Figure 9 and Sample A and Sample B shown in Figure 10 are enzymatically modified isoquercitrin (IQC-G(mix)) with reduced isoquercitrin (IQC). Oral administration of Sample 2 results in higher in vivo absorbability than when orally administering Sample 1, while oral administration of Sample B results in lower in vivo absorbability than when orally administering Sample A. These results suggest that when the total amount of IQC-G (4≤) is 15 mol% or less, and the total amount of IQC-G (1-3) including G3 is 60 mol% or more, the in vivo absorbability will be higher than that of conventional enzymatically modified isoquercitrin (Sample 1 or A).

Further, combining the results shown in Figure 1 and Figure 10 indicates that amylase treatment or the like of enzymatically modified isoquercitrin (IQC-G(mix)) reduces the total proportion of IQC-G(4≤) and relatively increases the total proportion of IQC-G(1-3), thus enhancing in vivo absorbability. As amylase treatment proceeds and G(4≤) disappears, decomposition of G3 subsequently starts, and the total proportion of IQC-G3 is thus reduced. Therefore, under the condition that the total proportion of IQC-G1 and that of IQC-4(≤) are constant, the larger the total proportion of IQC-G3, the higher the in vivo absorbability, and accordingly, the in vivo absorbability decreases with the progression of the amylase reaction.

Example 1: Tablet

| | (Wt%) |
|---|---|
| IQC-G (1-3) fraction (Preparation Example 2) | 18 |
| Lactose | 78 |
| Sucrose fatty acid ester | 4 |

The above components were uniformly mixed to give tablets each weighing 250 mg.

Example 2: Powder or Granules

| | (Wt%) |
|---|---|
| IQC-G(1-3) fraction (Preparation Example 2) | 18 |
| Lactose | 60 |
| Starch | 22 |

The above components were uniformly mixed to give a powder or granules.

Example 3: Capsule product

| | (Wt%) |
|---|---|
| Gelatin | 70.0 |
| Glycerol | 22.9 |
| Methyl parahydroxybenzoate | 0.15 |
| Propyl parahydroxybenzoate | 0.35 |
| Water | Remaining |
| Total | 100.00% |

Soft capsules formed from the above components was filled with the granules prepared in Example 2 by an ordinary method, giving soft capsule products each weighing 250 mg.

Example 4: Drink

| | | |
|---|---|---|
| Taste component: | Sodium dl-tartrate | 0.10 g |
| | Succinic acid | 0.009 g |
| Sweet component: Sugar syrup | | 800.00 g |
| Sour taste component: Citric acid | | 12.00 g |
| Vitamin C | | 10.00 g |
| IQC-G(1-3) fraction (Preparation Example 2) | | 1.80 g |
| Vitamin E | | 30.00 g |
| Cyclodextrin | | 5.00 g |
| Flavoring | | 15.00 ml |
| Potassium chloride | | 1.00 g |
| Magnesium sulfate | | 0.50 g |

The above components were mixed, and water was added thereto to make 10 L. This drink was prepared so that the dose at one administration would be about 250 ml.

Example 5: Candy

| | |
|---|---|
| Sugar | 98 g |
| Starch syrup (Brix 75) | 91 g |
| Concentrate of an IQC-G(1-3) fraction (Preparation Example 2) (Brix 40) | 75g |

The above components were fully mixed and boiled down to a moisture content of 2%, giving candies each weighing 2 g.

## Claims

**1.** A quercetin glycoside composition comprising a mixture of quercetin glycosides represented by the following formula:

[Formula 1]

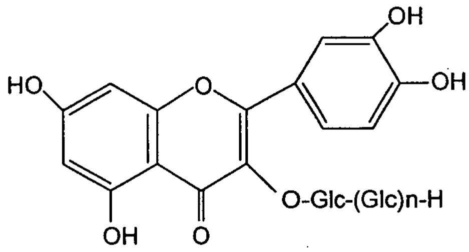

wherein Glc represents a glucose residue; and n is 0 or a positive integer of 1 or more, the quercetin glycoside composition comprising at least a quercetin glycoside wherein n is 3, and satisfying the following requirement (a):

(a) the composition comprises a mixture of quercetin glycosides in which n is 3, and in which other n values may be 1 or 2, or 1 and 2, in a total proportion of 50 mol% or more, and quercetin glycosides wherein n is 4 or more in a total proportion of 15 mol% or less.

**2.** The quercetin glycoside composition of Claim 1, wherein the total proportion of quercetin glycosides wherein n is 4 or more is 10 mol% or less.

**3.** The quercetin glycoside composition of Claim 1, wherein the total proportion of quercetin glycosides in which n is 3, and in which other n values may be 1 or 2, or 1 and 2, is 60 mol% or more.

**4.** The quercetin glycoside composition of Claim 1, wherein the total proportion of quercetin glycosides in which n is 3, and in which other n values may be 1 or 2, or 1 and 2, is 70 mol% or more.

**5.** The quercetin glycoside composition of Claim 1, further satisfying at least one of the following requirements (b) and (c):

(b) the composition contains a quercetin glycoside wherein n is 0 in 20 mol% or less, and
(c) the composition comprises a mixture of 2 types of quercetin glycosides wherein n is 2, and wherein n is 3, and the total proportion thereof is 50 mol% or more.

**6.** The quercetin glycoside composition of Claim 1, prepared by treating an enzymatically modified isoquercitrin with amylase.

**7.** The quercetin glycoside composition of Claim 6, wherein the amylase is β-amylase.

**8.** A food product containing the quercetin glycoside composition of Claim 1.

**9.** A method for preparing the quercetin glycoside composition of Claim 1 having a higher orally administered in vivo absorbability than an enzymatically modified isoquercitrin, the method comprising a step of reducing a proportion of quercetin glycosides represented by the' following formula:

[Formula 2]

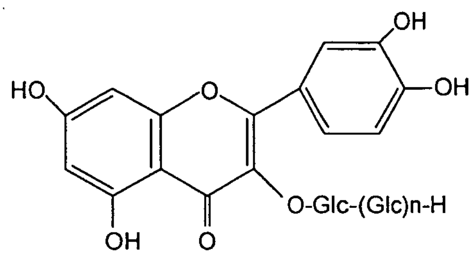

wherein Glc represents a glucose residue, n is an integer of 4 or more, so as to make a total proportion thereof 15 mol% or less.

**10.** The method of Claim 9, wherein the step of reducing the proportion of quercetin glycosides represented by the formula includes treatment of an enzymatically modified isoquercitrin with amylase.

**11.** The method of Claim 10, wherein the amylase is β-amylase.

**12.** A method for enhancing orally administered in vivo absorbability of quercetin glycoside composition, comprising, using an enzymatically modified isoquercitrin as a starting material, a step of reducing a proportion of quercetin glycosides represented by the following formula:

[Formula 3]

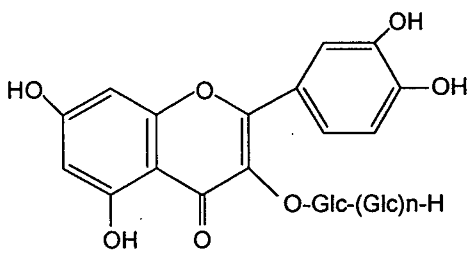

wherein Glc represents a glucose residue, and n is an integer of 4 or more.

**13.** The method of Claim 12, wherein the step of reducing the proportion of quercetin glycosides represented by the formula includes treatment of the enzymatically modified isoquercitrin with amylase.

**14.** The method of Claim 13, wherein the amylase is β-amylase.

**15.** The method of Claim 12, further comprising a step of reducing a proportion of isoquercitrin represented by the following formula:

[Formula 4]

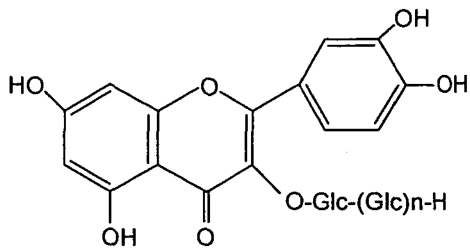

wherein Glc represents a glucose residue, and n is 0.

FIG. 1

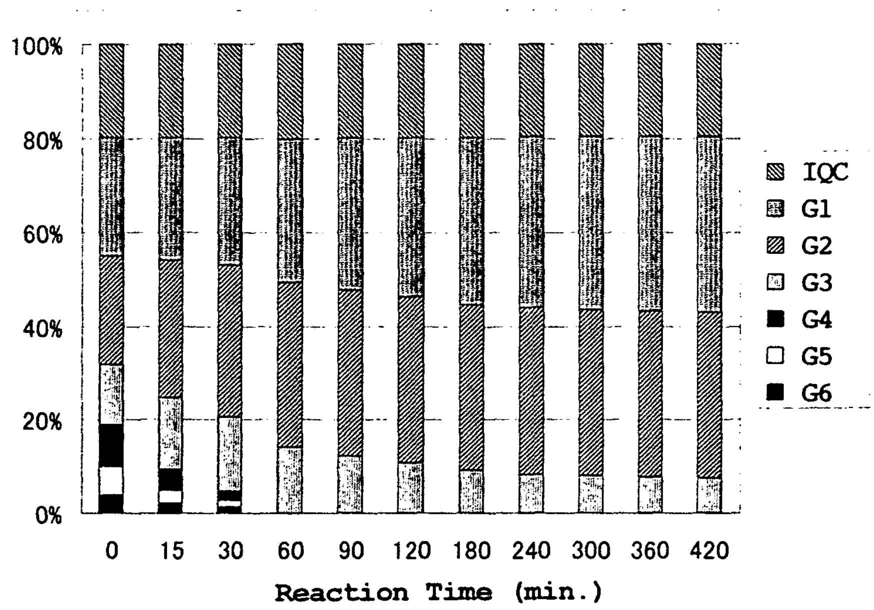
100%
80%
60%
40%
20%
0%
0
15
30
60
90
120
180
240
300
360
420
Reaction Time (min.)
IQC
G1
G2
G3
G4
G5
G6

FIG. 2

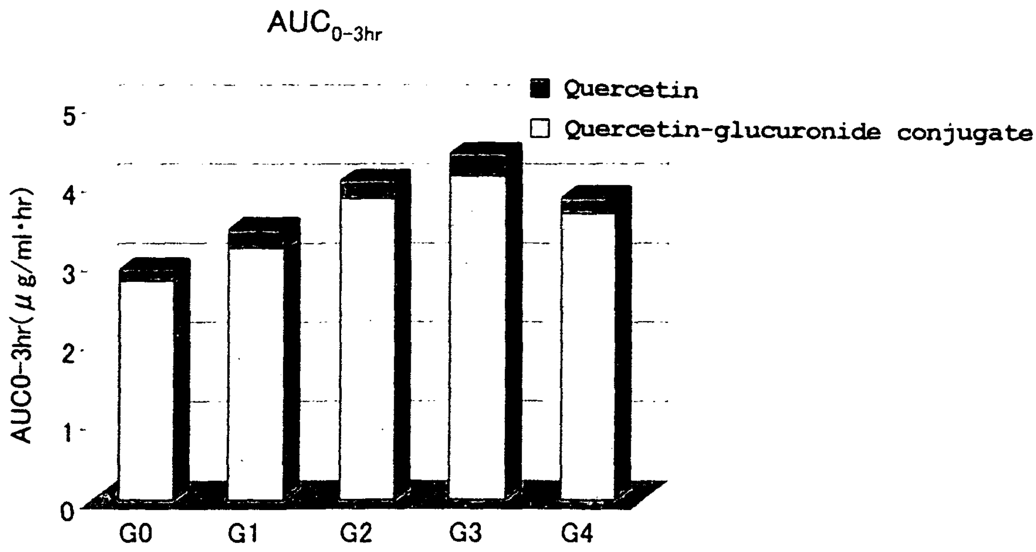
AUC0-3hr
Quercetin
Quercetin-glucuronide conjugate
AUC0-3hr(μg/ml·hr)
0
1
2
3
4
5
G0
G1
G2
G3
G4

FIG.3

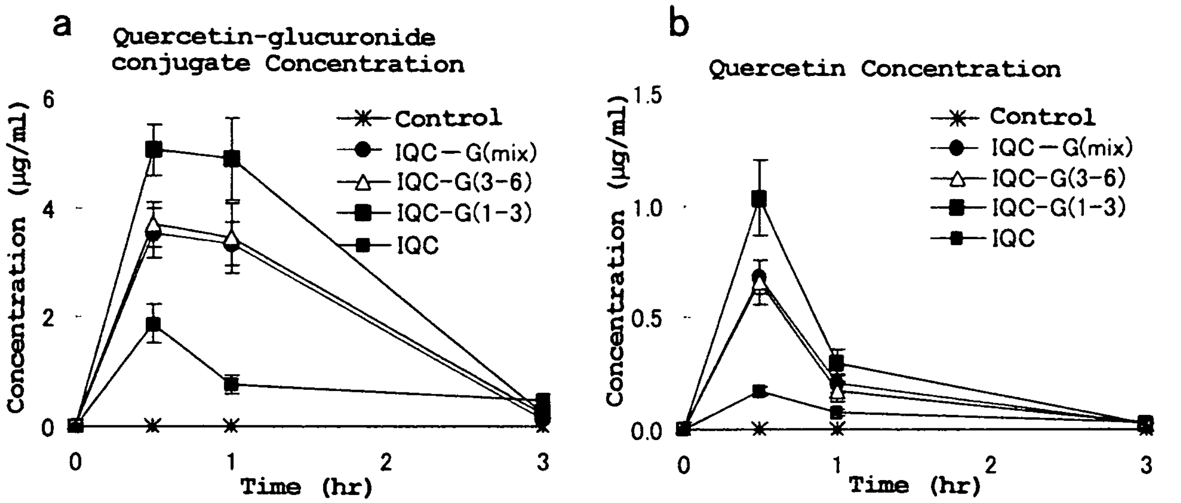
a
Quercetin-glucuronide conjugate Concentration
Concentration (μg/ml)
0
2
4
6
Time (hr)
0
1
2
3
Control
IQC−G(mix)
IQC-G(3-6)
IQC-G(1-3)
IQC
b
Quercetin Concentration
Concentration (μg/ml)
0.0
0.5
1.0
1.5
Time (hr)
0
1
2
3
Control
IQC−G(mix)
IQC-G(3-6)
IQC-G(1-3)
IQC

FIG.4

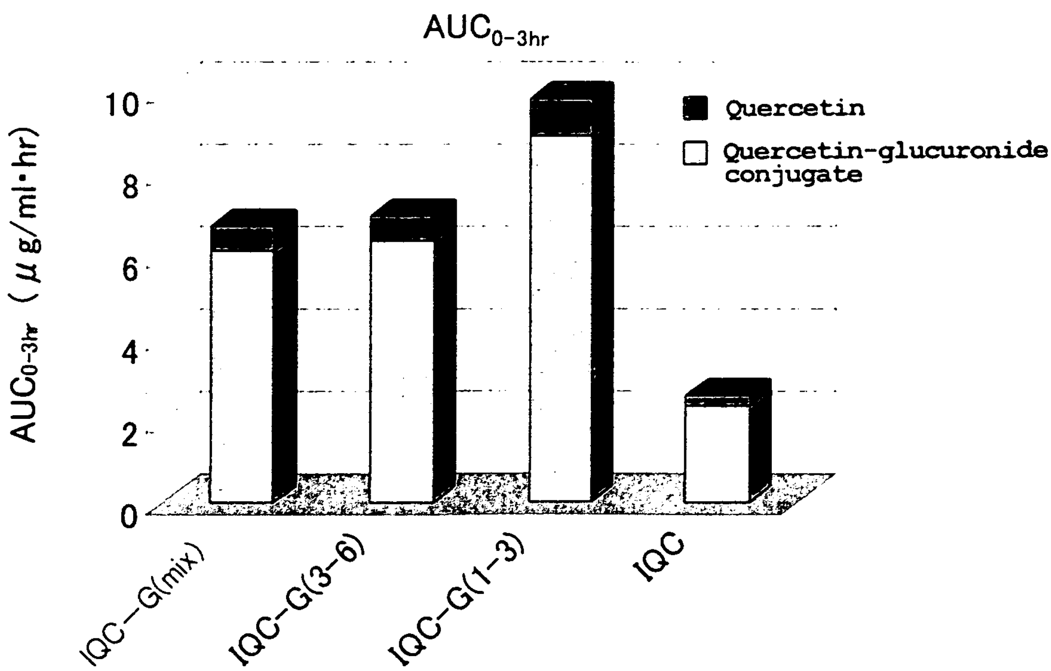
AUC0-3hr
AUC0-3hr (μg/ml·hr)
0
2
4
6
8
10
Quercetin
Quercetin-glucuronide conjugate
IQC−G(mix)
IQC-G(3-6)
IQC-G(1-3)
IQC

FIG. 5

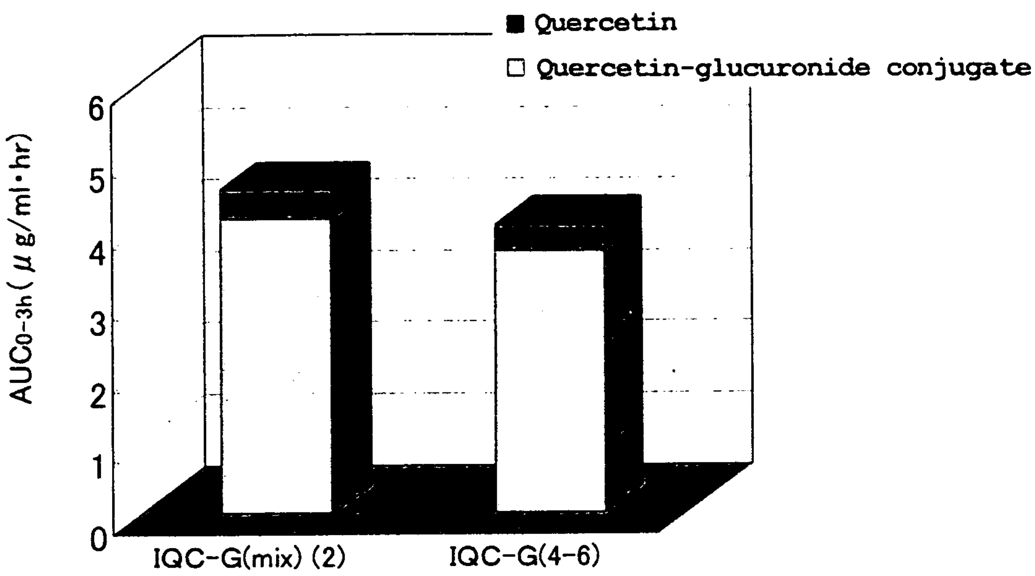
Quercetin
Quercetin-glucuronide conjugate
AUC0-3h (μg/ml·hr)
6
5
4
3
2
1
0
IQC-G(mix) (2)
IQC-G(4-6)

FIG. 6

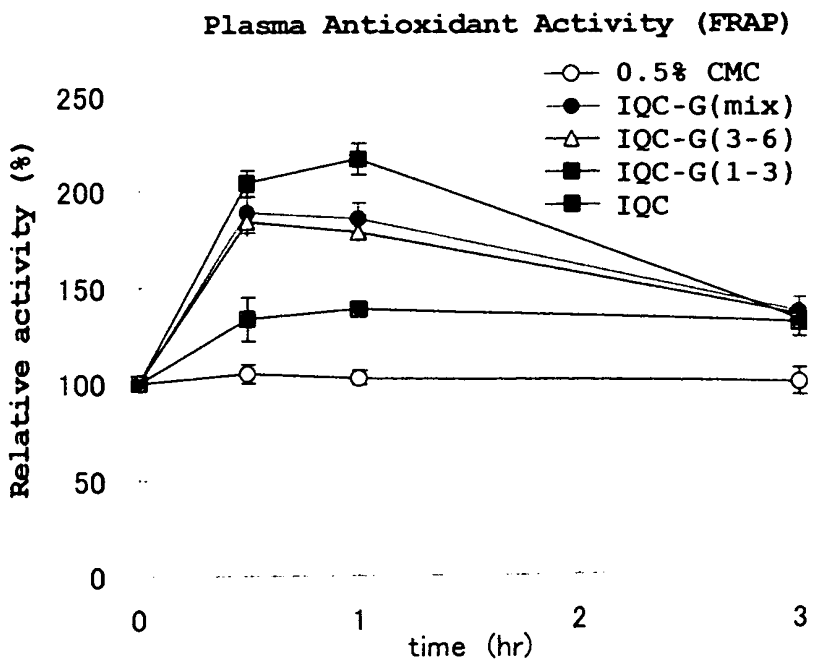
Plasma Antioxidant Activity (FRAP)
0.5% CMC
IQC-G(mix)
IQC-G(3-6)
IQC-G(1-3)
IQC
Relative activity (%)
250
200
150
100
50
0
0
1
2
3
time (hr)

FIG. 7

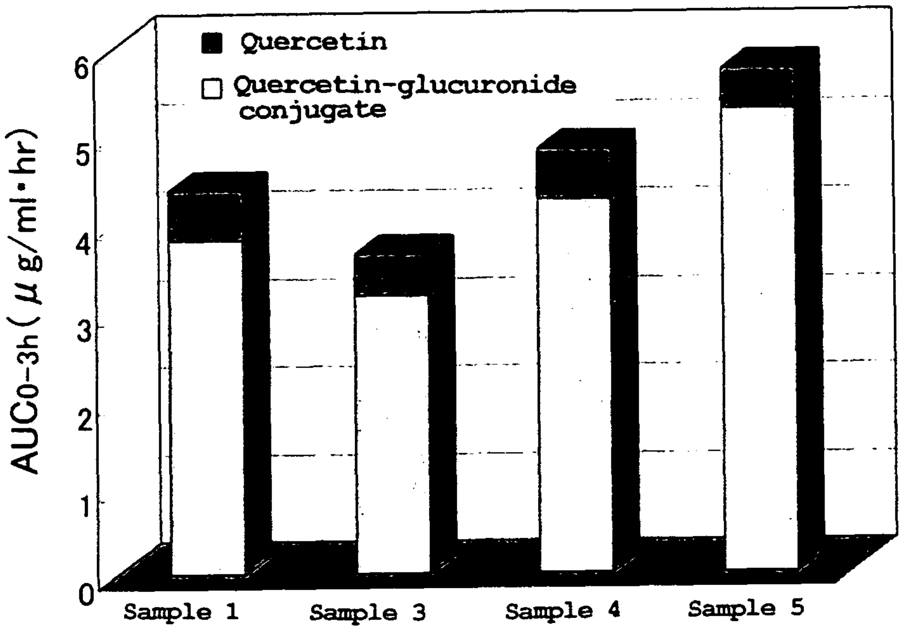
Quercetin
Quercetin-glucuronide conjugate
AUC0-3h (μg/ml·hr)
0
1
2
3
4
5
6
Sample 1
Sample 3
Sample 4
Sample 5

FIG. 8

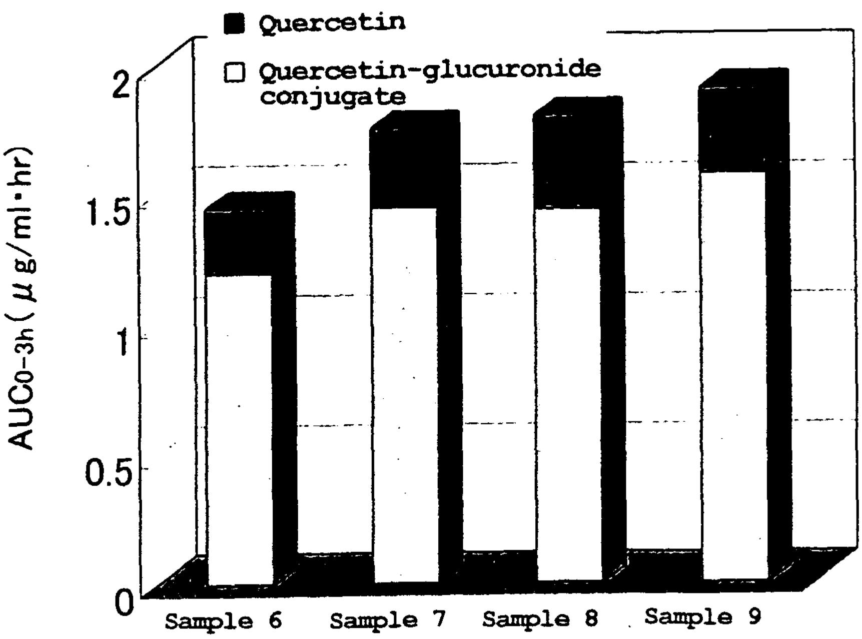
Quercetin
Quercetin-glucuronide conjugate
AUC0-3h (μg/ml·hr)
0
0.5
1
1.5
2
Sample 6
Sample 7
Sample 8
Sample 9

FIG.9

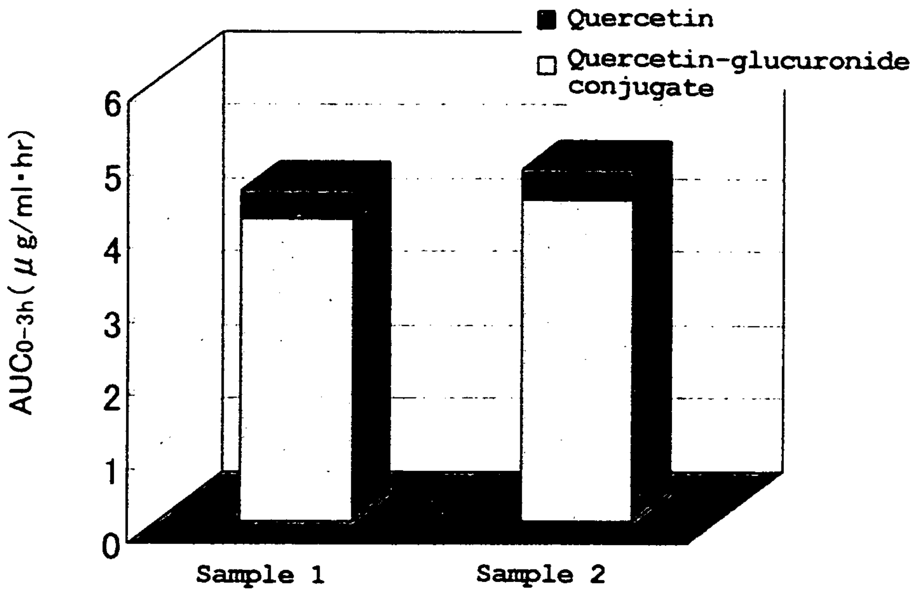
Quercetin
Quercetin-glucuronide conjugate
AUC0-3h (μg/ml·hr)
0
1
2
3
4
5
6
Sample 1
Sample 2

FIG.10

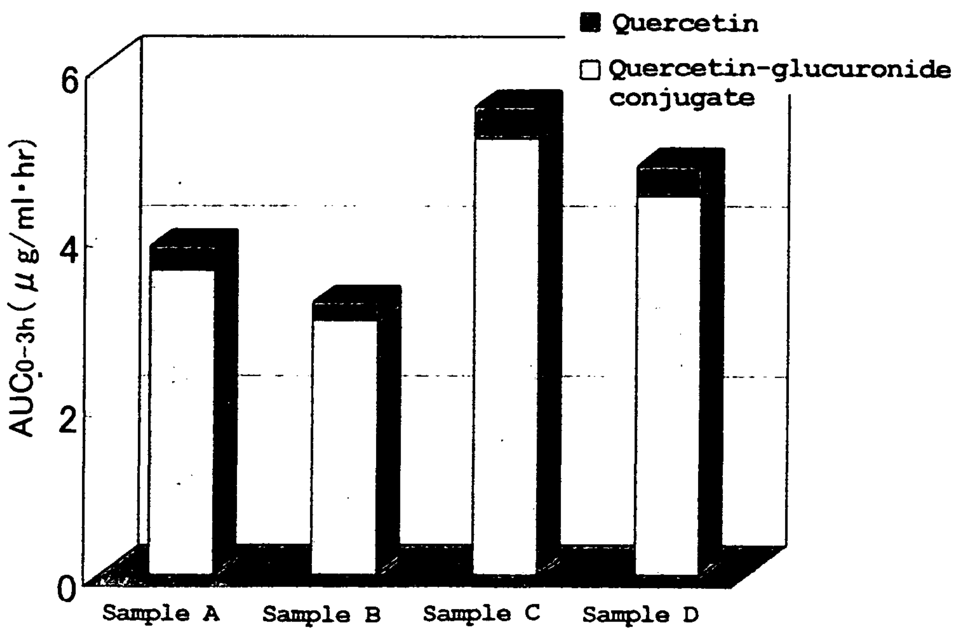
Quercetin
Quercetin-glucuronide conjugate
AUC0-3h (μg/ml·hr)
0
2
4
6
Sample A
Sample B
Sample C
Sample D

## INTERNATIONAL SEARCH REPORT

International application No.
PCT/JP2005/024113

A. CLASSIFICATION OF SUBJECT MATTER
*C12P19/22*(2006.01), *C07H17/07*(2006.01), *A23L1/29*(2006.01), *A23L2/38*(2006.01), *A23G3/34*(2006.01), *A61K31/7048*(2006.01), *A61P39/06*(2006.01)

According to International Patent Classification (IPC) or to both national classification and IPC

B. FIELDS SEARCHED

Minimum documentation searched (classification system followed by classification symbols)
*C12P19/22*(2006.01), *C07H17/07*(2006.01), *A23L1/29*(2006.01), *A23L2/38*(2006.01), *A23G3/34*(2006.01), *A61K31/7048*(2006.01), *A61P39/06*(2006.01)

Documentation searched other than minimum documentation to the extent that such documents are included in the fields searched

Electronic data base consulted during the international search (name of data base and, where practicable, search terms used)
CA/BIOSIS/MEDLINE/WPIDS(STN)

C. DOCUMENTS CONSIDERED TO BE RELEVANT

| Category* | Citation of document, with indication, where appropriate, of the relevant passages | Relevant to claim No. |
|---|---|---|
| A | AKIYAMA Takumi et al. "Constituents of Enzymatically Modified Isoquercitrin and Enzymatically Modified Rutin (Extract)" 2000, Shokuhin Eiseigaku Zasshi, Vol.41(1), pages 54 to 60, full text | 1-15 |
| A | JP 9-94077 A (San-Ei Gen F.F.I., Inc.), 08 April, 1997 (08.04.97), Full text (Family: none) | 1-15 |
| A | JP 3-255013 A (San-Ei Chemical Industries,Ltd.), 13 November, 1991 (13.11.91), Full text (Family: none) | 1-15 |

☐ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 March, 2006 (28.03.06) | 04 April, 2006 (04.04.06) |
| Name and mailing address of the ISA/ Japanese Patent Office | Authorized officer |
| Facsimile No. | Telephone No. |

Form PCT/ISA/210 (second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2000078956 A
- JP 2004059522 A
- JP 1213293 A


### Non-patent literature cited in the description


- **SHIMOI K. et al.** *J. Agric. Food Chem.,* 2003, vol. 51, 2785-2789
- **MIDDLTON EJ. et al.** *Pharmacol. Rev.,* 2000, vol. 52, 673-751
- **HOLLMAN PC. et al.** *Arch Toxicol Suppl.,* 1998, vol. 20, 237-248
- **MORAND C. et al.** *Free Rad Res.,* 2000, vol. 33, 667-676
- *FFI Journal,* 2004, vol. 209 (7), 622-628
- *Syokuhin Eisei Gaku Zasshi,* vol. 41 (1), 54-60
- **IRIS F F BENZIE et al.** *Analytical Biochemistry,* 1996, vol. 239, 70-76